# EUROPEAN PATENT APPLICATION

(11) **EP 4 400 121 A1**
(43) Date of publication of application: **17.07.2024**
(21) Application number: 22864711.1
(22) Date of filing: 02.09.2022
(51) Int. Cl.: A61K 47/68, A61K 38/08, A61K 39/395, A61P 35/00, A61P 35/02, C07K 7/06, C07K 16/28, C07K 16/30, C07K 16/46, C07K 19/00, C12N 15/13, C12N 15/62, C12P 21/08

(54) **PHARMACEUTICAL COMPOSITION FOR TREATING AND/OR PREVENTING CANCER**

(30) Priority: 03.09.2021 JP 2021143764
(71) Applicant: Toray Industries, Inc., Tokyo 103-8666 (JP)
(72) Inventor: OKANO, Fumiyoshi, Kamakura-shi, Kanagawa 248-8555 (JP); SAITO, Takanori, Kamakura-shi, Kanagawa 248-8555 (JP)
(74) Representative: Mewburn Ellis LLP
(86) International application number: PCT/JP2022/033055
(87) International publication number: WO 2023/033129

(57) **Abstract**

Dolastatin 10 or a derivative thereof is linked to an antibody or a fragment thereof having immunological reactivity against a CAPRIN-1 protein having an amino acid sequence represented by any of even-numbered SEQ ID NOs among SEQ ID NOs: 2 to 30 or an amino acid sequence having 80% or more sequence identity to the amino acid sequence, thereby enhancing the antitumor effect of the antibody or the fragment thereof.

## Description

### Technical Field

The present invention relates to a conjugate of an antibody against a CAPRIN-1 protein and dolastatin 10 or a derivative thereof, and medical use thereof for treatment and/or prevention of a cancer.

### Background Art

Various antibody drugs targeting specific antigenic proteins on cancer cells are applied as therapeutic drugs for cancers with fewer adverse reactions to cancer treatment because of their cancer specificity. For example, cytoplasmic-activation and proliferation-associated protein 1 (CAPRIN-1) is expressed on the cell membrane surface of many solid cancers, and antibodies against this CAPRIN-1 protein have been known to be promising in medical use for the treatment and/or prevention of cancers (Patent Literature 1).

Dolastatin 10, an antitumor pentapeptide that inhibits microtubule polymerization and thus cell division by mitosis, was isolated from the Indian Ocean sea hare *Dolabella auricularia* (Non Patent Literature 1). After dolastatin 10 was obtained and its structure was elucidated, many derivatives of dolastatin 10 with antitumor properties have been synthesized, and some dolastatin derivatives have been developed and put into practical use as chemotherapeutic agents against cancer.

In recent years, studies have been made to enhance the pharmacological effects of the antibody drugs against cancers. Particularly, antibody-drug conjugates (ADCs) in which an antibody is conjugated with a drug having the strong ability to directly kill cells have been actively developed (Non Patent Literatures 2 and 3). Although there are few successful cases of ADCs using dolastatin 10 derivatives, PADCETM^{(R)} (Enfortumab vedotin-ejfv), which is one of the dolastatin 10 derivatives linked to an antibody against Nectin-4, has a response rate of 50% or more for advanced or metastatic urothelial carcinoma that is cisplatin-unresponsive (Non Patent Literature 4).

### Citation List

### Patent Literature

### Patent Literature 1: WO2010/016526

### Non Patent Literature

Non Patent Literature 1: Springer Chemistry 1997 (ISSN0071-7886)
Non Patent Literature 2: Lancet Oncol. 2016; 17: e256-62
Non Patent Literature 3: Pharm Res. 2015 Nov; 32 (11): 3526-40
Non Patent Literature 4: Ther. Adv. Urol. 2020 vol. 12: 1-10

### Summary of Invention

### Technical Problem

As mentioned above, ADCs utilizing derivatives of dolastatin 10 have already been put into practical use, but successful cases are limited. The antitumor effects strong enough to completely regress various cancers have not yet been obtained.

Therefore, an object of the present invention is to enhance the antitumor effect of an ADC using dolastatin 10 or a derivative thereof.

### Solution to Problem

The present inventor has conducted diligent studies and consequently completed the present invention by finding that: a conjugate of an antibody or a fragment thereof against a CAPRIN-1 protein and dolastatin 10 or a derivative thereof exerts a much stronger antitumor effect than that of the antibody against the CAPRIN-1 protein or the fragment thereof used alone; and the effect of enhancing the antitumor effect by conjugating the antibody against the CAPRIN-1 protein or the fragment thereof to the dolastatin 10 or the derivative thereof is much superior to the effect of enhancing the antitumor effect by conjugating an existing antibody drug for a cancer to the dolastatin 10 or the derivative thereof.

Specifically, the present invention has the following features (1) to (14).

(1) A conjugate of an antibody or a fragment thereof linked to dolastatin 10 or a derivative thereof, wherein the antibody or the fragment thereof has immunological reactivity against a CAPRIN-1 protein having an amino acid sequence represented by any of even-numbered SEQ ID NOs among SEQ ID NOs: 2 to 30 or an amino acid sequence having 80% or more sequence identity to the amino acid sequence.
(2) The conjugate according to (1), wherein the antibody or the fragment thereof has immunological reactivity against a partial polypeptide of the CAPRIN-1 protein, wherein the partial polypeptide has an amino acid sequence represented by any of SEQ ID NOs: 31 to 35 and 296 to 299, 308, and 309 or an amino acid sequence having 80% or more sequence identity to the amino acid sequence.
(3) The conjugate according to (1) or (2), wherein the antibody is a monoclonal antibody or a polyclonal antibody.
(4) The conjugate according to any of (1) to (3), wherein the antibody or the fragment thereof is any of the following (A) to (M):
   (A) an antibody or a fragment thereof comprising a heavy chain variable region comprising complementarity-determining regions of SEQ ID NOs: 36, 37, and 38 (CDR1, CDR2, and CDR3, respectively), and a light chain variable region comprising complementarity-determining regions of SEQ ID NOs: 40, 41, and 42 (CDR1, CDR2, and CD3, respectively), and having immunological reactivity against the CAPRIN-1 protein,
   (B) an antibody or a fragment thereof comprising a heavy chain variable region comprising complementarity-determining regions of SEQ ID NOs: 44, 45, and 46 (CDR1, CDR2, and CDR3, respectively), and a light chain variable region comprising complementarity-determining regions of SEQ ID NOs: 48, 49, and 50 (CDR1, CDR2, and CDR3, respectively), and having immunological reactivity against the CAPRIN-1 protein,
   (C) an antibody or a fragment thereof comprising a heavy chain variable region comprising complementarity-determining regions of SEQ ID NOs: 52, 53, and 54 (CDR1, CDR2, and CDR3, respectively), and a light chain variable region comprising complementarity-determining regions of SEQ ID NOs: 56, 57, and 58 (CDR1, CDR2, and CDR3, respectively), and having immunological reactivity against the CAPRIN-1 protein,
   (D) an antibody or a fragment thereof comprising a heavy chain variable region comprising complementarity-determining regions of SEQ ID NOs: 60, 61, and 62 (CDR1, CDR2, and CDR3, respectively), and a light chain variable region comprising complementarity-determining regions of SEQ ID NOs: 64, 65, and 66 (CDR1, CDR2, and CDR3, respectively), and having immunological reactivity against the CAPRIN-1 protein,
   (E) an antibody or a fragment thereof comprising a heavy chain variable region comprising complementarity-determining regions of SEQ ID NOs: 170, 171, and 172 (CDR1, CDR2, and CDR3, respectively), and a light chain variable region comprising complementarity-determining regions of SEQ ID NOs: 173, 174, and 175 (CDR1, CDR2, and CDR3, respectively), and having immunological reactivity against the CAPRIN-1 protein,
   (F) an antibody or a fragment thereof comprising a heavy chain variable region comprising complementarity-determining regions of SEQ ID NOs: 176, 177, and 178 (CDR1, CDR2, and CDR3, respectively), and a light chain variable region comprising complementarity-determining regions of SEQ ID NOs: 179, 180, and 181 (CDR1, CDR2, and CDR3, respectively), and having immunological reactivity against the CAPRIN-1 protein,
   (G) an antibody or a fragment thereof comprising a heavy chain variable region comprising complementarity-determining regions of SEQ ID NOs: 182, 183, and 184 (CDR1, CDR2, and CDR3, respectively), and a light chain variable region comprising complementarity-determining regions of SEQ ID NOs: 185, 186, and 187 (CDR1, CDR2, and CDR3, respectively), and having immunological reactivity against the CAPRIN-1 protein,
   (H) an antibody or a fragment thereof comprising a heavy chain variable region comprising complementarity-determining regions of SEQ ID NOs: 188, 189, and 190 (CDR1, CDR2, and CDR3, respectively), and a light chain variable region comprising complementarity-determining regions of SEQ ID NOs: 191, 192, and 193 (CDR1, CDR2, and CDR3, respectively), and having immunological reactivity against the CAPRIN-1 protein,
   (I) an antibody or a fragment thereof comprising a heavy chain variable region comprising complementarity-determining regions of SEQ ID NOs: 146, 147, and 148 (CDR1, CDR2, and CDR3, respectively), and a light chain variable region comprising complementarity-determining regions of SEQ ID NOs: 149, 150, and 151 (CDR1, CDR2, and CDR3, respectively), and having immunological reactivity against the CAPRIN-1 protein,
   (J) an antibody or a fragment thereof comprising a heavy chain variable region comprising complementarity-determining regions of SEQ ID NOs: 272, 273, and 274 (CDR1, CDR2, and CDR3, respectively), and a light chain variable region comprising complementarity-determining regions of SEQ ID NOs: 275, 276, and 277 (CDR1, CDR2, and CDR3, respectively), and having immunological reactivity against the CAPRIN-1 protein,
   (K) an antibody or a fragment thereof comprising a heavy chain variable region comprising complementarity-determining regions of SEQ ID NOs: 290, 291, and 292 (CDR1, CDR2, and CDR3, respectively), and a light chain variable region comprising complementarity-determining regions of SEQ ID NOs: 293, 294, and 295 (CDR1, CDR2, and CDR3, respectively), and having immunological reactivity against the CAPRIN-1 protein,
   (L) an antibody or a fragment thereof comprising a heavy chain variable region comprising complementarity-determining regions of SEQ ID NOs: 301, 302, and 303 (CDR1, CDR2, and CDR3, respectively), and a light chain variable region comprising complementarity-determining regions of SEQ ID NOs: 305, 306, and 307 (CDR1, CDR2, and CDR3, respectively), and having immunological reactivity against the CAPRIN-1 protein, and
   (M) an antibody or a fragment thereof comprising a heavy chain variable region comprising complementarity-determining regions of SEQ ID NOs: 134, 135, and 136 (CDR1, CDR2, and CDR3, respectively), and a light chain variable region comprising complementarity-determining regions of SEQ ID NOs: 137, 138, and 139 (CDR1, CDR2, and CDR3, respectively), and having immunological reactivity against the CAPRIN-1 protein.
(5) The conjugate according to any of (1) to (4), wherein the antibody or the fragment thereof is any of the following (a) to (al):
   (a) an antibody or a fragment thereof, comprising a heavy chain variable region comprising the amino acid sequence of SEQ ID NO: 39 and a light chain variable region comprising the amino acid sequence of SEQ ID NO: 43,
   (b) an antibody or a fragment thereof, comprising a heavy chain variable region comprising the amino acid sequence of SEQ ID NO: 47 and a light chain variable region comprising the amino acid sequence of SEQ ID NO: 51,
   (c) an antibody or a fragment thereof, comprising a heavy chain variable region comprising the amino acid sequence of SEQ ID NO: 55 and a light chain variable region comprising the amino acid sequence of SEQ ID NO: 59,
   (d) an antibody or a fragment thereof, comprising a heavy chain variable region comprising the amino acid sequence of SEQ ID NO: 63 and a light chain variable region comprising the amino acid sequence of SEQ ID NO: 67,
   (e) an antibody or a fragment thereof, comprising a heavy chain variable region comprising the amino acid sequence of SEQ ID NO: 68 and a light chain variable region comprising the amino acid sequence of SEQ ID NO: 69,
   (f) an antibody or a fragment thereof, comprising a heavy chain variable region comprising the amino acid sequence of SEQ ID NO: 70 and a light chain variable region comprising the amino acid sequence of SEQ ID NO: 71,
   (g) an antibody or a fragment thereof, comprising a heavy chain variable region comprising the amino acid sequence of SEQ ID NO: 72 and a light chain variable region comprising the amino acid sequence of SEQ ID NO: 73,
   (h) an antibody or a fragment thereof, comprising a heavy chain variable region comprising the amino acid sequence of SEQ ID NO: 74 and a light chain variable region comprising the amino acid sequence of SEQ ID NO: 75,
   (i) an antibody or a fragment thereof, comprising a heavy chain variable region comprising the amino acid sequence of SEQ ID NO: 76 and a light chain variable region comprising the amino acid sequence of SEQ ID NO: 77,
   (j) an antibody or a fragment thereof, comprising a heavy chain variable region comprising the amino acid sequence of SEQ ID NO: 78 and a light chain variable region comprising the amino acid sequence of SEQ ID NO: 79,
   (k) an antibody or a fragment thereof, comprising a heavy chain variable region comprising the amino acid sequence of SEQ ID NO: 80 and a light chain variable region comprising the amino acid sequence of SEQ ID NO: 81,
   (l) an antibody or a fragment thereof, comprising a heavy chain variable region comprising the amino acid sequence of SEQ ID NO: 82 and a light chain variable region comprising the amino acid sequence of SEQ ID NO: 83,
   (m) an antibody or a fragment thereof, comprising a heavy chain variable region comprising the amino acid sequence of SEQ ID NO: 84 and a light chain variable region comprising the amino acid sequence of SEQ ID NO: 85,
   (n) an antibody or a fragment thereof, comprising a heavy chain variable region comprising the amino acid sequence of SEQ ID NO: 86 and a light chain variable region comprising the amino acid sequence of SEQ ID NO: 87,
   (o) an antibody or a fragment thereof, comprising a heavy chain variable region comprising the amino acid sequence of SEQ ID NO: 88 and a light chain variable region comprising the amino acid sequence of SEQ ID NO: 89,
   (p) an antibody or a fragment thereof, comprising a heavy chain variable region comprising the amino acid sequence of SEQ ID NO: 90 and a light chain variable region comprising the amino acid sequence of SEQ ID NO: 91,
   (q) an antibody or a fragment thereof, comprising a heavy chain variable region comprising the amino acid sequence of SEQ ID NO: 92 and a light chain variable region comprising the amino acid sequence of SEQ ID NO: 93,
   (r) an antibody or a fragment thereof, comprising a heavy chain variable region comprising the amino acid sequence of SEQ ID NO: 94 and a light chain variable region comprising the amino acid sequence of SEQ ID NO: 95,
   (s) an antibody or a fragment thereof, comprising a heavy chain variable region comprising the amino acid sequence of SEQ ID NO: 96 and a light chain variable region comprising the amino acid sequence of SEQ ID NO: 97,
   (t) an antibody or a fragment thereof, comprising a heavy chain variable region comprising the amino acid sequence of SEQ ID NO: 98 and a light chain variable region comprising the amino acid sequence of SEQ ID NO: 99,
   (u) an antibody or a fragment thereof, comprising a heavy chain variable region comprising the amino acid sequence of SEQ ID NO: 100 and a light chain variable region comprising the amino acid sequence of SEQ ID NO: 101,
   (v) an antibody or a fragment thereof, comprising a heavy chain variable region comprising the amino acid sequence of SEQ ID NO: 102 and a light chain variable region comprising the amino acid sequence of SEQ ID NO: 103,
   (w) an antibody or a fragment thereof, comprising a heavy chain variable region comprising the amino acid sequence of SEQ ID NO: 104 and a light chain variable region comprising the amino acid sequence of SEQ ID NO: 105,
   (x) an antibody or a fragment thereof, comprising a heavy chain variable region comprising the amino acid sequence of SEQ ID NO: 106 and a light chain variable region comprising the amino acid sequence of SEQ ID NO: 107,
   (y) an antibody or a fragment thereof, comprising a heavy chain variable region comprising the amino acid sequence of SEQ ID NO: 108 and a light chain variable region comprising the amino acid sequence of SEQ ID NO: 109,
   (z) an antibody or a fragment thereof, comprising a heavy chain variable region comprising the amino acid sequence of SEQ ID NO: 110 and a light chain variable region comprising the amino acid sequence of SEQ ID NO: 111,
   (aa) an antibody or a fragment thereof, comprising a heavy chain variable region comprising the amino acid sequence of SEQ ID NO: 112 and a light chain variable region comprising the amino acid sequence of SEQ ID NO: 113,
   (ab) an antibody or a fragment thereof, comprising a heavy chain variable region comprising the amino acid sequence of SEQ ID NO: 114 and a light chain variable region comprising the amino acid sequence of SEQ ID NO: 115,
   (ac) an antibody or a fragment thereof, comprising a heavy chain variable region comprising the amino acid sequence of SEQ ID NO: 116 and a light chain variable region comprising the amino acid sequence of SEQ ID NO: 117,
   (ad) an antibody or a fragment thereof, comprising a heavy chain variable region comprising the amino acid sequence of SEQ ID NO: 118 and a light chain variable region comprising the amino acid sequence of SEQ ID NO: 119,
   (ae) an antibody or a fragment thereof, comprising a heavy chain variable region comprising the amino acid sequence of SEQ ID NO: 120 and a light chain variable region comprising the amino acid sequence of SEQ ID NO: 121,
   (at) an antibody or a fragment thereof, comprising a heavy chain variable region comprising the amino acid sequence of SEQ ID NO: 122 and a light chain variable region comprising the amino acid sequence of SEQ ID NO: 123,
   (ag) an antibody or a fragment thereof, comprising a heavy chain variable region comprising the amino acid sequence of SEQ ID NO: 124 and a light chain variable region comprising the amino acid sequence of SEQ ID NO: 125,
   (ah) an antibody or a fragment thereof, comprising a heavy chain variable region comprising the amino acid sequence of SEQ ID NO: 126 and a light chain variable region comprising the amino acid sequence of SEQ ID NO: 127,
   (ai) an antibody or a fragment thereof, comprising a heavy chain variable region comprising the amino acid sequence of SEQ ID NO: 128 and a light chain variable region comprising the amino acid sequence of SEQ ID NO: 129,
   (aj) an antibody or a fragment thereof, comprising a heavy chain variable region comprising the amino acid sequence of SEQ ID NO: 130 and a light chain variable region comprising the amino acid sequence of SEQ ID NO: 131,
   (ak) an antibody or a fragment thereof, comprising a heavy chain variable region comprising the amino acid sequence of SEQ ID NO: 132 and a light chain variable region comprising the amino acid sequence of SEQ ID NO: 133, and
   (al) an antibody or a fragment thereof, comprising a heavy chain variable region comprising the amino acid sequence of SEQ ID NO: 300 and a light chain variable region comprising the amino acid sequence of SEQ ID NO: 304.
(6) The conjugate according to any of (1) to (5), wherein the antibody is a human antibody, a humanized antibody, a chimeric antibody, or a single-chain antibody.
(7) The conjugate according to any of (1) to (6), wherein the antibody or the fragment thereof is linked to the dolastatin 10 or the derivative thereof via a linker.
(8) The conjugate according to any of (1) to (7), wherein the dolastatin 10 derivative is monomethyl auristatin E (MMAE), monomethyl auristatin F (MMAF), or a derivative thereof.
(9) A pharmaceutical composition for treatment and/or prevention of a cancer, comprising the conjugate according to any of (1) to (8) as an active ingredient.
(10) The pharmaceutical composition according to (9), further comprising one type or two or more types of antitumor agents together or separately in combination.
(11) The pharmaceutical composition according to (9) or (10), wherein the cancer is a cancer expressing a CAPRIN-1 protein on the cell membrane surface.
(12) The pharmaceutical composition according to any of (9) to (11), wherein the cancer is breast cancer, kidney cancer, pancreatic cancer, colon cancer, lung cancer, brain tumor, gastric cancer, uterine cancer, ovarian cancer, prostate cancer, bladder cancer, esophageal cancer, leukemia, lymphoma, liver cancer, gallbladder cancer, bile duct cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, urothelial carcinoma, renal cell carcinoma, colorectal cancer, gastroesophageal junction cancer, hepatocellular carcinoma, glioblastoma, primary central nervous system lymphoma, primary testicular lymphoma, biliary tract cancer, sarcoma, fibrosarcoma, basal cell carcinoma, Paget's disease, or skin cancer.
(13) A method for treating and/or preventing a cancer, comprising administering the conjugate according to any of (1) to (8) to a subject.
(14) A method for treating and/or preventing a cancer, comprising administering the conjugate according to any of (1) to (8) and one type or two or more types of antitumor agents together or separately in combination to a subject.

### Advantageous Effects of Invention

The conjugate according to the present invention not only exerts a much stronger antitumor effect than that of an antibody or a fragment thereof against a CAPRIN-1 protein used alone but also is superior in antitumor effect to a known conjugate of an antibody drug for a cancer and dolastatin 10 or a derivative thereof. In addition, the effect of enhancing the antitumor effect by conjugating the antibody against the CAPRIN-1 protein or the fragment thereof to the dolastatin 10 or the derivative thereof is much superior to the effect of enhancing the antitumor effect by conjugating an existing antibody drug for a cancer to the dolastatin 10 or the derivative thereof. Thus, the conjugate according to the present invention is effective for the treatment or prevention of a cancer.

### Description of Embodiments

The conjugate of an antibody or a fragment thereof against a CAPRIN-1 protein (hereinafter, referred to as an "anti-CAPRIN-1 antibody") and dolastatin 10 or a derivative thereof used in the present invention can be evaluated for its antitumor activity, as mentioned later, by examining *in vivo* the inhibition of tumor growth in a cancer-bearing animal.

In the present invention, the "conjugate" refers to an antibody linked to dolastatin 10 or a derivative thereof via a covalent bond. The linking between the antibody and the dolastatin 10 or the derivative thereof may be done by a linker.

The anti-CAPRIN-1 antibody that is a constituent of the conjugate according to the present invention may be a monoclonal antibody or a polyclonal antibody but is preferably a monoclonal antibody. The anti-CAPRIN-1 antibody may also be any type of antibody as long as the conjugate of the present invention can exert antitumor activity, and may be a recombinant antibody, a human antibody, a humanized antibody, a chimeric antibody, or a non-human animal antibody.

Dolastatin 10 or a derivative thereof, which forms the conjugate according to the present invention, is known as one of the substances having toxicity to cancer cells by the action of inhibiting the formation of the microtubules or mitosis by directly acting on microtubules.

The subject who is the target to be treated and/or prevented for cancer according to the present invention is a mammal such as a human, a pet animal, livestock, or a sport animal, and a preferred subject is a human.

Hereinafter, the anti-CAPRIN-1 antibody, the dolastatin 10 or the derivative thereof, the conjugate of the anti-CAPRIN-1 antibody and the dolastatin 10 or the derivative thereof, the pharmaceutical composition comprising the conjugate, and the method for treating and/or preventing a cancer using the conjugate, according to the present invention will be described.

### <Anti-CAPRIN-1 antibody>

Among CAPRIN-1 proteins having an amino acid sequence represented by any of even-numbered SEQ ID NOs among SEQ ID NOs: 2 to 30 and having immunological reactivity against the anti-CAPRIN-1 antibody used in the present invention, the amino acid sequences represented by SEQ ID NOs: 6, 8, 10, 12, and 14 are the amino acid sequences of canine CAPRIN-1 proteins; the amino acid sequence represented by SEQ ID NOs: 2 and 4 are the amino acid sequences of human CAPRIN-1 proteins; the amino acid sequence represented by SEQ ID NO: 16 is the amino acid sequence of a bovine CAPRIN-1 protein; the amino acid sequence represented by SEQ ID NO: 18 is the amino acid sequence of an equine CAPRIN-1 protein; the amino acid sequences represented by SEQ ID NOs: 20 to 28 are the amino acid sequences of mouse CAPRIN-1 proteins; and the amino acid sequence represented by SEQ ID NO: 30 is the amino acid sequence of a chicken CAPRIN-1 protein.

In addition, the anti-CAPRIN-1 antibody used in the present invention may have immunological reactivity against a variant of the CAPRIN-1 protein having 80% or more, preferably 90% or more, more preferably 95% or more, further preferably 99% or more sequence identity to the amino acid sequence represented by any of even-numbered SEQ ID NOs among SEQ ID NOs: 2 to 30. In this context, the term "% sequence identity" means a percentage (%) of the number of identical amino acids (or bases) to the total number of amino acids (or bases) when two sequences are aligned so that the maximum degree of similarity can be achieved with or without introducing a gap.

In the present invention, the anti-CAPRIN-1 antibody that is used for preparing the conjugate means an antibody or a fragment thereof having immunological reactivity against a full-length CAPRIN-1 protein or a fragment thereof. In this context, the "immunological reactivity" means the property of the antibody binding to the CAPRIN-1 protein or a partial polypeptide thereof *in vivo.*

The anti-CAPRIN-1 antibody used in the present invention may be a monoclonal antibody or a polyclonal antibody.

The polyclonal antibody having immunological reactivity against the full-length CAPRIN-1 protein or the fragment thereof (anti-CAPRIN-1 polyclonal antibody) can be obtained, for example, by immunizing a mouse, a human antibody-producing mouse, a rat, a rabbit, a chicken, or the like with a naturally occurring CAPRIN-1 protein, or a fusion protein thereof with GST or the like, or a partial peptide thereof and obtaining serum therefrom, and applying the obtained serum to ammonium sulfate precipitation, protein A, protein G, a DEAE ion-exchange column, an affinity column linked to a CAPRIN-1 protein or a partial peptide, or the like.

As for the full-length CAPRIN-1 protein or the fragment thereof to be used in the immunization, the base sequences and amino acid sequences of CAPRIN-1 and homologs thereof are available, for example, by accessing GenBank (NCBI, USA) and using an algorithm such as BLAST or FASTA (Karlin and Altschul, Proc. Natl. Acad. Sci. USA, 90: 5873-5877, 1993; and Altschul et al., Nucleic Acids Res. 25: 3389-3402, 1997). In addition, a method for preparing the CAPRIN-1 protein is available with reference to WO2014/012479, or can be carried out using, for example, cells expressing the CAPRIN-1 protein.

The monoclonal antibody having immunological reactivity against the full-length CAPRIN-1 protein or the fragment thereof (anti-CAPRIN-1 monoclonal antibody) can be obtained, for example, by: administering SK-BR-3 (breast cancer cells expressing CAPRIN-1) or the full-length CAPRIN-1 protein or the fragment thereof, or the like to a mouse for immunization; fusing spleen cells separated from the mouse with myeloma cells; and selecting a clone producing anti-CAPRIN-1 monoclonal antibodies from among the obtained fusion cells (hybridomas). The antibody produced from the hybridoma thus selected can be prepared in the same way as the method for purifying the polyclonal antibody mentioned above.

The antibody used in the present invention includes human antibodies, humanized antibodies, chimeric antibodies, and non-human animal antibodies.

The human antibody can be obtained by: sensitizing EB virus-infected human lymphocytes with the protein, protein-expressing cells, or lysates thereof; fusing the sensitized lymphocytes with human-derived myeloma cells such as U266 cells; and obtaining an antibody having immunological reactivity against the full-length CAPRIN-1 protein or the fragment thereof from the obtained fusion cells.

The humanized antibody, also called reshaped human antibody, is an engineered antibody. The humanized antibody is constructed by grafting complementarity-determining regions of an antibody derived from an immunized animal onto complementarity-determining regions of a human antibody. A genetic engineering technique commonly used for constructing humanized antibodies is also well-known. Specifically, for example, DNA sequences designed to link complementarity-determining regions of a mouse or rabbit antibody, to framework regions of a human antibody are synthesized by PCR from several prepared oligonucleotides having overlapping terminal portions. The obtained DNAs are ligated with DNAs encoding human antibody constant regions. The resulting ligation products are incorporated into expression vectors, which are then transferred to hosts for antibody production to obtain the antibody of interest (see, EP239400 and WO96/02576). The framework regions of a human antibody connected via the complementarity-determining regions are selected so that the complementarity-determining regions form a favorable antigen-binding site. If necessary, an amino acid in the framework regions of antibody variable regions may be substituted so that the complementarity-determining regions of a reshaped human antibody form an appropriate antigen-binding site (Sato K. et al., Cancer Research 1993, 53: 851-856). In addition, these framework regions may be replaced with framework regions derived from various human antibodies (see WO99/51743).

Antibodies are typically heteromultimeric glycoproteins each comprising at least two heavy chains and two light chains. The antibodies are each composed of two identical light chains and two identical heavy chains. Each heavy chain has a heavy chain variable region at one end, followed by a series of constant regions. Each light chain has a light chain variable region at one end, followed by a series of constant regions. The variable regions contain certain variable regions called complementarity-determining regions (CDRs) and impart binding specificity to the antibody. Portions relatively conserved in the variable regions are called framework regions (FRs). The complete heavy chain and light chain variable regions each contain four FRs connected via three CDRs (CDR1 to CDR3).

The sequences of human-derived heavy chain and light chain constant regions and variable regions are available from NCBI (USA; GenBank, UniGene, etc.). For example, the following sequences may be referred to: Accession No. J00228 for a human IgG1 heavy chain constant region; Accession No. J00230 for a human IgG2 heavy chain constant region; Accession Nos. V00557, X64135, X64133, etc., for a human light chain κ constant region; and Accession Nos. X64132, X64134, etc., for a human light chain λ constant region.

A chimeric antibody is an antibody prepared from a combination of sequences derived from different animals and may be, for example, an antibody composed of heavy chain variable region and light chain variable region of a mouse antibody and heavy chain constant region and light chain constant region of a human antibody. The chimeric antibody can be prepared using a method known in the art and is obtained, for example, by: ligating DNAs encoding the antibody V regions to DNAs encoding the human antibody C regions; incorporating the resulting ligation products into expression vectors; and transferring the vectors into hosts for antibody production.

The non-human animal antibody is obtained by immunizing an animal with a sensitizing antigen according to a method known in the art and, as a general method, by intraperitoneally, intracutaneously, or subcutaneously injecting a sensitizing antigen to an animal such as a mouse. For the injection of the sensitizing antigen, the antigen is mixed in an appropriate amount with various adjuvants including CFA (complete Freund's adjuvant), and the mixture is administered to the animal a plurality of times. The animal is immunized and then verified to contain anti-CAPRIN-1 antibodies in serum. The serum can be obtained and applied, as mentioned above, to ammonium sulfate precipitation, protein A, protein G, a DEAE ion-exchange column, an affinity column bound with a CAPRIN-1 protein or a partial peptide, or the like to obtain the non-human animal antibody. In the case of obtaining a monoclonal antibody from a non-human animal, immune cells can be collected from an immunized animal and subjected to cell fusion with myeloma cells to obtain the monoclonal antibody. The cell fusion of the immune cells with the myeloma cells may be carried out according to a method known in the art (see Kohler, G. and Milstein, C. Methods Enzymol. (1981) 73, 3-46).

The antibody used in the present invention may also be obtained as a recombinant antibody produced using a genetic engineering technique by cloning genes of the antibody from a hybridoma; incorporating the antibody genes into appropriate vectors; and transferring the vectors into hosts (see Carl, A.K., Borrebaeck, James, W. Larrick, THERAPEUTIC MONOCLONAL ANTIBODIES, Published in the United Kingdom by MACMILLAN PUBLISHERS LTD, 1990).

The anti-CAPRIN-1 antibody that is used for obtaining the conjugate of the present invention may be an antibody in which an amino acid in a variable region (e.g., FR) or a constant region is substituted with another amino acid. The amino acid substitution is the substitution of one or more, for example, less than 15, less than 10, 8 or less, 6 or less, 5 or less, 4 or less, 3 or less, or 2 or less amino acids, preferably 1 to 9 amino acids. The substituted antibody should be an antibody that has the property of specifically binding to the antigen and binding affinity for the antigen equivalent to or higher than those of the corresponding unsubstituted antibody and causes no rejection when applied to humans.

The anti-CAPRIN-1 antibody used in the present invention is expected to have a stronger antitumor effect when it has the higher binding affinity for the CAPRIN-1 protein on cancer cell surface. Its association constant (affinity constant) Ka (kon/koff) is preferably at least 10⁷ M⁻¹, at least 10⁸ M⁻¹, at least 5 x 10⁸ M⁻¹, at least 10⁹ M⁻¹, at least 5 x 10⁹ M⁻¹, at least 10¹⁰ M⁻¹, at least 5 x 10¹⁰ M⁻¹, at least 10¹¹ M⁻¹, at least 5 x 10¹¹ M⁻¹, at least 10¹² M⁻¹, or at least 10¹³ M⁻¹.

The binding activity of the anti-CAPRIN-1 antibody used in the present invention against effector cells may be improved by substituting 1, 2 or several amino acids in the heavy chain constant region of the antibody or by removing fucose linked to N-acetylglucosamine in a N-glycoside-linked sugar chain attached to the heavy chain constant region. Such an antibody may only have the amino acid substitution or may be in a composition comprising a fucosylated antibody.

The antibody in which 1, 2 or several amino acids in the heavy chain constant region are substituted may be prepared with reference to, for example, WO2004/063351, WO2011/120135, U.S. Patent No. 8388955, WO2011/005481, U.S. Patent No. 6737056, and/or WO2005/063351.

The antibody lacking fucose added to N-acetylglucosamine in a N-glycoside-linked sugar chain in the heavy chain constant region has been removed, or cells producing the antibody, may be prepared with reference to U.S. Patent No. 6602684, European Patent No. 1914244, and/or U.S. Patent No. 7579170. A composition of the antibody lacking fucose added to N-acetylglucosamine in a N-glycoside-linked sugar chain attached to the heavy chain constant region, and the antibody having the fucose, or cells producing the composition may be prepared with reference to, for example, U.S. Patent No. 8642292.

Methods for preparing and purifying the anti-CAPRIN-1 polyclonal antibody, the anti-CAPRIN-1 monoclonal antibody, and the antibody used in the present invention, and a method for preparing the CAPRIN-1 protein or the partial polypeptide thereof to be used in immunization may be carried out with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212.

Specific examples of the anti-CAPRIN-1 antibody according to the present invention include anti-CAPRIN-1 antibodies disclosed in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 mentioned above. Preferred examples of the anti-CAPRIN-1 antibody include the following.

An antibody or a fragment thereof having immunological reactivity against the amino acid sequence represented by SEQ ID NO: 2 or SEQ ID NO: 4 or a partial polypeptide of the CAPRIN-1 protein having an amino acid sequence having 80% or more (preferably 85% or more, more preferably 90% or more, further preferably 95% or more, still further preferably 99% or more) sequence identity to the amino acid sequence represented by SEQ ID NO: 2 or SEQ ID NO: 4.

An antibody or a fragment thereof having immunological reactivity against a partial polypeptide of the CAPRIN-1 protein having the amino acid sequence represented by SEQ ID NO: 31 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 36, 37, and 38 (CDR1, CDR2, and CDR3, respectively), and a light chain variable region comprising complementarity-determining regions of SEQ ID NOs: 40, 41, and 42 (CDR1, CDR2, and CDR3, respectively), and having immunological reactivity against the CAPRIN-1 protein; an antibody or a fragment thereof which comprises a heavy chain variable region comprising complementarity-determining regions of SEQ ID NOs: 140, 141, and 142 (CDR1, CDR2, and CDR3, respectively), and a light chain variable region comprising complementarity-determining regions of SEQ ID NOs: 143, 144, and 145 (CDR1, CDR2, and CDR3, respectively), and having immunological reactivity against the CAPRIN-1 protein; or an antibody or a fragment thereof which comprises a heavy chain variable region comprising complementarity-determining regions of SEQ ID NOs: 164, 165, and 166 (CDR1, CDR2, and CDR3, respectively), and a light chain variable region comprising complementarity-determining regions of SEQ ID NOs: 167, 168, and 169 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 39 and a light chain variable region comprising the amino acid sequence of SEQ ID NO: 43; an antibody or a fragment thereof comprising a heavy chain variable region comprising the amino acid sequence of SEQ ID NO: 70 and a light chain variable region comprising the amino acid sequence of SEQ ID NO: 71; or an antibody or a fragment thereof comprising a heavy chain variable region comprising the amino acid sequence of SEQ ID NO: 78 and a light chain variable region comprising the amino acid sequence of SEQ ID NO: 79.

An antibody or a fragment thereof having immunological reactivity against a partial polypeptide of the CAPRIN-1 protein having the amino acid sequence represented by SEQ ID NO: 33 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 60, 61, and 62 (CDR1, CDR2, and CDR3, respectively), and a light chain variable region comprising complementarity-determining regions of SEQ ID NOs: 64, 65, and 66 (CDR1, CDR2, and CDR3, respectively), and having immunological reactivity against the CAPRIN-1 protein; more preferably an antibody or a fragment thereof comprising a heavy chain variable region comprising the amino acid sequence of SEQ ID NO: 63 and a light chain variable region comprising the amino acid sequence of SEQ ID NO: 67.

An antibody or a fragment thereof having immunological reactivity against a partial polypeptide of the CAPRIN-1 protein having the amino acid sequence represented by SEQ ID NO: 32 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 52, 53, and 54 (CDR1, CDR2, and CDR3, respectively), and a light chain variable region comprising complementarity-determining regions of SEQ ID NOs: 56, 57, and 58 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 55 and a light chain variable region comprising the amino acid sequence of SEQ ID NO: 59.

An antibody or a fragment thereof having immunological reactivity against a partial polypeptide of the CAPRIN-1 protein having the amino acid sequence represented by SEQ ID NO: 34 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 170, 171, and 172 (CDR1, CDR2, and CDR3, respectively), and a light chain variable region comprising complementarity-determining regions of SEQ ID NOs: 173, 174, and 175 (CDR1, CDR2, and CDR3, respectively), and having immunological reactivity against the CAPRIN-1 protein; or an antibody or a fragment thereof which comprises a heavy chain variable region comprising complementarity-determining regions of SEQ ID NOs: 176, 177, and 178 (CDR1, CDR2, and CDR3, respectively), and a light chain variable region comprising complementarity-determining regions of SEQ ID NOs: 179, 180, and 181 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 80 and a light chain variable region comprising the amino acid sequence of SEQ ID NO: 81; or an antibody or a fragment thereof comprising a heavy chain variable region comprising the amino acid sequence of SEQ ID NO: 82 and a light chain variable region comprising the amino acid sequence of SEQ ID NO: 83.

An antibody or a fragment thereof having immunological reactivity against a partial polypeptide of the CAPRIN-1 protein having the amino acid sequence represented by SEQ ID NO: 35 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 182, 183, and 184 (CDR1, CDR2, and CDR3, respectively), and a light chain variable region comprising complementarity-determining regions of SEQ ID NOs: 185, 186, and 187 (CDR1, CDR2, and CDR3, respectively), and having immunological reactivity against the CAPRIN-1 protein; or an antibody or a fragment thereof which comprises a heavy chain variable region comprising complementarity-determining regions of SEQ ID NOs: 188, 189, and 190 (CDR1, CDR2, and CDR3, respectively), and a light chain variable region comprising complementarity-determining regions of SEQ ID NOs: 191, 192, and 193 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 84 and a light chain variable region comprising the amino acid sequence of SEQ ID NO: 85; or an antibody or a fragment thereof comprising a heavy chain variable region comprising the amino acid sequence of SEQ ID NO: 86 and a light chain variable region comprising the amino acid sequence of SEQ ID NO: 87.

An antibody or a fragment thereof which comprises a heavy chain variable region comprising complementarity-determining regions of SEQ ID NOs: 44, 45, and 46 (CDR1, CDR2, and CDR3, respectively), and a light chain variable region comprising complementarity-determining regions of SEQ ID NOs: 48, 49, and 50 (CDR1, CDR2, and CDR3, respectively), and having immunological reactivity against the CAPRIN-1 protein. Preferably, an antibody or a fragment thereof comprising a heavy chain variable region comprising the amino acid sequence of SEQ ID NO: 47 and a light chain variable region comprising the amino acid sequence of SEQ ID NO: 51.

An antibody or a fragment thereof having immunological reactivity against a partial polypeptide of the CAPRIN-1 protein having the amino acid sequence represented by SEQ ID NO: 296 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 146, 147, and 148 (CDR1, CDR2, and CDR3, respectively), and a light chain variable region comprising complementarity-determining regions of SEQ ID NOs: 149, 150, and 151 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 72 and a light chain variable region comprising the amino acid sequence of SEQ ID NO: 73.

An antibody or a fragment thereof having immunological reactivity against a partial polypeptide of the CAPRIN-1 protein having the amino acid sequence represented by SEQ ID NO: 297 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 272, 273, and 274 (CDR1, CDR2, and CDR3, respectively), and a light chain variable region comprising complementarity-determining regions of SEQ ID NOs: 275, 276, and 277 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 114 and a light chain variable region comprising the amino acid sequence of SEQ ID NO: 115.

An antibody or a fragment thereof having immunological reactivity against a partial polypeptide of the CAPRIN-1 protein having the amino acid sequence represented by SEQ ID NO: 298 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 290, 291, and 292 (CDR1, CDR2, and CDR3, respectively), and a light chain variable region comprising complementarity-determining regions of SEQ ID NOs: 293, 294, and 295 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 120 and a light chain variable region comprising the amino acid sequence of SEQ ID NO: 121.

An antibody or a fragment thereof having immunological reactivity against a partial polypeptide of the CAPRIN-1 protein having the amino acid sequence represented by SEQ ID NO: 299 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 301, 302, and 303 (CDR1, CDR2, and CDR3, respectively), and a light chain variable region comprising complementarity-determining regions of SEQ ID NOs: 305, 306, and 307 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 300 and a light chain variable region comprising the amino acid sequence of SEQ ID NO: 304.

An antibody or a fragment thereof having immunological reactivity against a partial polypeptide of the CAPRIN-1 protein having the amino acid sequence represented by SEQ ID NO: 308 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 134, 135, and 136 (CDR1, CDR2, and CDR3, respectively), and a light chain variable region comprising complementarity-determining regions of SEQ ID NOs: 137, 138, and 139 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 68 and a light chain variable region comprising the amino acid sequence of SEQ ID NO: 69.

An antibody or a fragment thereof having immunological reactivity against a partial polypeptide of the CAPRIN-1 protein having the amino acid sequence represented by SEQ ID NO: 309 or an amino acid sequence having 80% or more (preferably 85% or more, more preferably 90% or more, further preferably 95% or more) sequence identity to the amino acid sequence. Preferably, an antibody or a fragment thereof which comprises a heavy chain variable region comprising complementarity-determining regions of SEQ ID NOs: 134, 135, and 136 (CDR1, CDR2, and CDR3, respectively), and a light chain variable region comprising complementarity-determining regions of SEQ ID NOs: 137, 138, and 139 (CDR1, CDR2, and CDR3, respectively), and having immunological reactivity against the CAPRIN-1 protein. More preferably, an antibody or a fragment thereof comprising a heavy chain variable region comprising the amino acid sequence of SEQ ID NO: 68 and a light chain variable region comprising the amino acid sequence of SEQ ID NO: 69.

Additionally, the following anti-CAPRIN-1 antibodies are preferably used.

An antibody or a fragment thereof comprising a heavy chain variable region comprising the amino acid sequence of SEQ ID NO: 68 and a light chain variable region comprising the amino acid sequence of SEQ ID NO: 69.

An antibody or a fragment thereof comprising a heavy chain variable region comprising the amino acid sequence of SEQ ID NO: 70 and a light chain variable region comprising the amino acid sequence of SEQ ID NO: 71.

An antibody or a fragment thereof comprising a heavy chain variable region comprising the amino acid sequence of SEQ ID NO: 72 and a light chain variable region comprising the amino acid sequence of SEQ ID NO: 73.

An antibody or a fragment thereof comprising a heavy chain variable region comprising the amino acid sequence of SEQ ID NO: 74 and a light chain variable region comprising the amino acid sequence of SEQ ID NO: 75.

An antibody or a fragment thereof comprising a heavy chain variable region comprising the amino acid sequence of SEQ ID NO: 76 and a light chain variable region comprising the amino acid sequence of SEQ ID NO: 77.

An antibody or a fragment thereof comprising a heavy chain variable region comprising the amino acid sequence of SEQ ID NO: 78 and a light chain variable region comprising the amino acid sequence of SEQ ID NO: 79.

An antibody or a fragment thereof comprising a heavy chain variable region comprising the amino acid sequence of SEQ ID NO: 80 and a light chain variable region comprising the amino acid sequence of SEQ ID NO: 81.

An antibody or a fragment thereof comprising a heavy chain variable region comprising the amino acid sequence of SEQ ID NO: 82 and a light chain variable region comprising the amino acid sequence of SEQ ID NO: 83.

An antibody or a fragment thereof comprising a heavy chain variable region comprising the amino acid sequence of SEQ ID NO: 84 and a light chain variable region comprising the amino acid sequence of SEQ ID NO: 85.

An antibody or a fragment thereof comprising a heavy chain variable region comprising the amino acid sequence of SEQ ID NO: 86 and a light chain variable region comprising the amino acid sequence of SEQ ID NO: 87.

An antibody or a fragment thereof comprising a heavy chain variable region comprising the amino acid sequence of SEQ ID NO: 88 and a light chain variable region comprising the amino acid sequence of SEQ ID NO: 89.

An antibody or a fragment thereof comprising a heavy chain variable region comprising the amino acid sequence of SEQ ID NO: 90 and a light chain variable region comprising the amino acid sequence of SEQ ID NO: 91.

An antibody or a fragment thereof comprising a heavy chain variable region comprising the amino acid sequence of SEQ ID NO: 92 and a light chain variable region comprising the amino acid sequence of SEQ ID NO: 93.

An antibody or a fragment thereof comprising a heavy chain variable region comprising the amino acid sequence of SEQ ID NO: 94 and a light chain variable region comprising the amino acid sequence of SEQ ID NO: 95.

An antibody or a fragment thereof comprising a heavy chain variable region comprising the amino acid sequence of SEQ ID NO: 96 and a light chain variable region comprising the amino acid sequence of SEQ ID NO: 97.

An antibody or a fragment thereof comprising a heavy chain variable region comprising the amino acid sequence of SEQ ID NO: 98 and a light chain variable region comprising the amino acid sequence of SEQ ID NO: 99.

An antibody or a fragment thereof comprising a heavy chain variable region comprising the amino acid sequence of SEQ ID NO: 100 and a light chain variable region comprising the amino acid sequence of SEQ ID NO: 101.

An antibody or a fragment thereof comprising a heavy chain variable region comprising the amino acid sequence of SEQ ID NO: 102 and a light chain variable region comprising the amino acid sequence of SEQ ID NO: 103.

An antibody or a fragment thereof comprising a heavy chain variable region comprising the amino acid sequence of SEQ ID NO: 104 and a light chain variable region comprising the amino acid sequence of SEQ ID NO: 105.

An antibody or a fragment thereof comprising a heavy chain variable region comprising the amino acid sequence of SEQ ID NO: 106 and a light chain variable region comprising the amino acid sequence of SEQ ID NO: 107.

An antibody or a fragment thereof comprising a heavy chain variable region comprising the amino acid sequence of SEQ ID NO: 108 and a light chain variable region comprising the amino acid sequence of SEQ ID NO: 109.

An antibody or a fragment thereof comprising a heavy chain variable region comprising the amino acid sequence of SEQ ID NO: 110 and a light chain variable region comprising the amino acid sequence of SEQ ID NO: 111.

An antibody or a fragment thereof comprising a heavy chain variable region comprising the amino acid sequence of SEQ ID NO: 112 and a light chain variable region comprising the amino acid sequence of SEQ ID NO: 113.

An antibody or a fragment thereof comprising a heavy chain variable region comprising the amino acid sequence of SEQ ID NO: 114 and a light chain variable region comprising the amino acid sequence of SEQ ID NO: 115.

An antibody or a fragment thereof comprising a heavy chain variable region comprising the amino acid sequence of SEQ ID NO: 116 and a light chain variable region comprising the amino acid sequence of SEQ ID NO: 117.

An antibody or a fragment thereof comprising a heavy chain variable region comprising the amino acid sequence of SEQ ID NO: 118 and a light chain variable region comprising the amino acid sequence of SEQ ID NO: 119.

An antibody or a fragment thereof comprising a heavy chain variable region comprising the amino acid sequence of SEQ ID NO: 120 and a light chain variable region comprising the amino acid sequence of SEQ ID NO: 121.

An antibody or a fragment thereof comprising a heavy chain variable region comprising the amino acid sequence of SEQ ID NO: 122 and a light chain variable region comprising the amino acid sequence of SEQ ID NO: 123.

An antibody or a fragment thereof comprising a heavy chain variable region comprising the amino acid sequence of SEQ ID NO: 124 and a light chain variable region comprising the amino acid sequence of SEQ ID NO: 125.

An antibody or a fragment thereof comprising a heavy chain variable region comprising the amino acid sequence of SEQ ID NO: 126 and a light chain variable region comprising the amino acid sequence of SEQ ID NO: 127.

An antibody or a fragment thereof comprising a heavy chain variable region comprising the amino acid sequence of SEQ ID NO: 128 and a light chain variable region comprising the amino acid sequence of SEQ ID NO: 129.

An antibody or a fragment thereof comprising a heavy chain variable region comprising the amino acid sequence of SEQ ID NO: 130 and a light chain variable region comprising the amino acid sequence of SEQ ID NO: 131.

An antibody or a fragment thereof comprising a heavy chain variable region comprising the amino acid sequence of SEQ ID NO: 132 and a light chain variable region comprising the amino acid sequence of SEQ ID NO: 133.

An antibody or a fragment thereof comprising a heavy chain variable region comprising the amino acid sequence of SEQ ID NO: 300 and a light chain variable region comprising the amino acid sequence of SEQ ID NO: 304.

In Examples described later, conjugates with a microtubule modulator were prepared using the polyclonal antibody or the monoclonal antibody against the full-length CAPRIN-1 protein or a polypeptide of a portion of an extracellular region expressed on the cell membrane surface of cancer cells, and verified to have a strong antitumor effect.

### <Dolastatin 10 or derivative thereof>

The dolastatin 10 or the derivative used in the present invention is not particularly limited as long as it is a compound directly acting on microtubules to inhibit the formation of the microtubules or mitosis, and is preferably a compound that inhibits microtubule polymerization (microtubule destabilizer) and has antitumor activity as described below.

Dolastatin 10 is a compound extracted from *Dolabella auricularia,* a mollusk in the family Aplysiidae of subclass opisthobranchia, and is a pentapeptide compound in which the amino acids dolavaline (Dov), valine (Val), dolaisoleucine (Dil), dolaproline (Dap), and dolaphenine (Doe) are bound and linked. The dolavaline site at the N-terminal side of dolastatin 10 is N,N-dimethylvaline.

The dolastatin 10 derivative is a compound in which a part of dolastatin 10 and the amino acids constituting the dolastatin 10 are substituted or deleted, a compound in which another amino acid is added to pentapeptide, or a compound in which the pentapeptide is modified with a substituent (substituted group). Specific examples thereof include a peptide compound in which valine (Val), dolaisoleuine (Dil), dolaproine (Dap), and dolaphenine (Doe) are linked, a peptide compound in which dolaisoleuine (Dil), dolaproine (Dap), and dolaphenine (Doe) are linked, and a compound in which a pyrrolidine ring of the dolaproline (Dap) moiety of dolastatin 10 is converted (e.g., CT-P26), and the dolastatin 10 derivative has antitumor activity as does dolastatin 10.

The dolastatin 10 derivative used in the present invention include the compounds described in "Pettit G. R. et al., Antineoplastic agents 337. Synthesis of dolastatin 10 structural modifications. 1995 Oct; 10 (7): 529-44", "Pettit G. R. et al. Med. Chem.", U.S. Patent No. 4,486,414, U.S. Patent No. 4,816,444, U.S. Patent No. 4,986,988, U.S. Patent No. 4,978,744, U.S. Patent No., U.S. Patent No., U.S. Patent No. 5,076,973, U.S. Patent No. 5,138,036, U.S. Patent No. 5,816,444, U.S. Patent No. 5,410,024, U.S. Patent No. 5,635,483, U.S. Patent No. 5,504,191, U.S. Patent No. 5,521,284, U.S. Patent No. 5,780,588, U.S. Patent No. 5,530,097, U.S. Patent No. 5,665,860, U.S. Patent No. 5,635,483, U.S. Patent No. 5,559,902, U.S. Patent No. 5,530,097, U.S. Patent No. 6,239,104, U.S. Patent No. 6,034,065, WO1994/013695, WO1993/023424, Japanese Patent Publication No. H9-77791, U.S. Patent No. 4,879,298, WO93/03054, WO95/09864, WO96/33212, U.S. Patent No. 5,840,699, WO2017/170637, WO2002/088172, U.S. Patent No. 6,884,869, U.S. Patent No. 7,098,308, U.S. Patent No. 7,256,257, U.S. Patent No. 7,423,116, WO2004/010957, U.S. Patent No. 7,659,241, U.S. Patent No. 7,659,241, U.S. Patent No. 7,851,437, U.S. Patent No. 8,906,376, WO2011/154359, U.S. Patent No. 8,722,629, WO2012/123423, U.S. Patent No. 9,029,406, European Patent No. 2686336, WO2005/081711, WO2006/132670, WO2007/008603, WO2012/123423, U.S. Patent No. 9,029,406, WO2012/143495, WO2012/143496, WO2012/143497, U.S. Patent No. 8,992,932, Japanese Patent No. 06250735, Japanese Patent No. 06088488, WO2019/164987 (Japanese Patent Publication No. 2021-513990), WO2005/081711, WO2007/008603, WO2011/1154359, WO2014/046441, WO1993/023424, WO1994/013684, WO1996/040751, WO1996/040752, WO1997/005162, WO1997/017364, WO1998/04278, WO1998/004581, WO1998/036765, WO1998/040400, WO1998/040092, WO1999/003879, WO1999/015130, WO2000/002906, WO2001/018032, WO2003/08378, WO2006/063707, WO2012/148943, WO2012/166559, WO2012/166560, WO2014/046441, WO2014/174060, WO2014/174062, WO2014/174064, WO2016/192527, WO2021/084532, WO2021/183542, CN109796522, CN110078787, US17/142169, and WO2003/008378.

The dolastatin 10 derivative preferably used in the present invention include a compound in which the thiazolephenethylamine at the C-terminal portion of dolastatin 10 is substituted with norephedrine (also referred to as monomethyl auristain E or MMAE), a compound in which the thiazolephenethylamine at the C-terminal portion of dolastatin 10 is substituted with phenylalanine (also referred to as monomethyl auristain For MMAF), and a derivatives thereof; aurlistatin PE (also referred to as YHI- 501 Sobidotin), a compound in which the C-terminal portion of dolastatin 10 is a phenylmethylamine; a compound having an amino group, a hydroxy group, a carboxylic acid, or a hydroxylamine at the C-terminal portion of dolastatin 10; and others such as PF-06380101, Tasidotin (also referred to as Synthadotin), auristatin PHE, auristatin PYE, anberstatin-269 (also referred to as Linked), N-demethyl-N-[4-(maleimidohexanohydorazido)-4-OxObutyl]auristatin W amide, BAY1168650 (also referred to as an auristatin W derivative), auristatin 0101, auristatin 2-AQ, auritatin 6-AQ, Aur 0101, monomethyl auristain F-hydroxypropylamide (MMAF-HPA), auristatin EB (AEB), auristatin EFP (AEFP), 5-benzoylvalerate-AE ester (AEVB), TZT-1027, soblidotin, CT-P26, and a derivative thereof. The dolastatin 10 derivative is preferably monomethyl auristain E (MMAE), monomethyl auristain F (MMAF), or a derivative thereof.

The dolastatin 10 derivative preferably used in the present invention is a compound having a functional group at the C-terminus of dolastatin 10 described in WO2017/170637, and preferably compounds shown in Tables 1 to 4. Specifically, the compounds include a compound having an ethyl ester obtained by a predetermined method using a dolastatin 10 derivative having a carboxyl group at the thiazole 4-position as an intermediate, a compound having an ethyl amide, a compound having an ethyl ester, a compound having adipic acid in a carbon chain form, a compound having succinic acid, a compound having PEG, and a compound having iminodiacetic acid. More specifically, the compounds include 2-((S-1-(((2S,3R)-3-((S)-1-((3R,4S,5 S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-mercaptoethyl)thiazole-4-carboxamide; 2-(tritylthio)ethyl 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxylate; 2-mercaptoethyl 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxylate; 2-((S)-1-((2R,3R)-3-((S)-1-((3R,4S,5 S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-methyl-N-(2-(trithylthio)ethyl)thiazole-4-carboxamide; 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-mercaptoethyl)-N-methylthiazole-4-carboxamide; 2-((tert-butoxycarbonyl)amino)ethyl 2-((S)-1-(((2S,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxylate; 2-aminoethyl 2-((S)-1-(((2S,3R)-3-((S)-1-((3R,4S,S S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxylate; tert-butyl (2-(2-((S-1-((2R,3R)-3-((S)-1-((3R,4S,5S)-4-((S)-2-((S)-2-(dimethylamino)-3-methylbutanamido)-N,3 -dimethylbutanamido)-3-methoxy-5-methylheptanoyl)pyrrolidin-2-yl)-3-methoxy-2-methylpropanamido)-2-phenylethyl)-N-methylthiazole-4-carboxamido)ethyl)carbamate; N-(2-aminoethyl)-2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-methylthiazole-4-carboxamide dihydrochloride; tert-butyl (2-(2-((S)-1-((2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-methylthiazole-4-carboxamido)ethyl)(methyl)carbamate; 2-((S)-1-((2R,3R)-3-((S)-1-((3R,4S,5S)-4-((S)-2-((S)-2-(dimethylamino)-3-methylbutanamido)-N,3 -dimethylbutanamido)-3-methoxy-5-methylheptanoyl)pyrrolidin-2-yl)-3-methoxy-2-methylpropanamido)-2-phenylethyl)-N-methyl-N-(2-(methylamino)ethyl)thiazole-4-carboxamide dihydrochloride; 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-hydroxyethyl)thiazole-4-carboxamide; 2-((tetrahydro-2H-pyran-2-yl)oxy)ethyl 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl) thiazole-4-carboxylate; 2-hydroxyethyl 2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxylate; 2-((S)-1-((2R,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-hydroxyethyl)-N-methylthiazole-4-carboxamide; N-(4-aminophenethyl)-2-((S)-1-(((2S,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxamide; tert-butyl (2-(2-((S)-1-((2R,3R)-3-((S)-1-((3R,4S,5 S)-4-((S)-2-((S)-2-(dimethylamino)-3-methylbutanamido)-N,3-dimaylbutanamido)-3-methoxy-5-methylheptanoyl)pyrrolidin-2-yl)-3-methoxy-2-methylpropanamido)-2-phenylethyl)thiazole-4-carboxamido)ethyl)carbamate; N-(2-aminoethyl)-2-((S)-1-(((2S,3R)-3-((S)-1-((3R,4S,SS)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxamide dihydrochloride; tert-butyl (2-(2-((S)-1-(((2S,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxamido)ethyl)(methyl)carbamate; 2-((S)-1-(((2S,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-(methylamino)ethyl)thiazole-4-carboxamide dihydrochloride; 2-((S)-1-(((2S,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(2-(3-(tritylthio)propanamido)ethyl)thiazole-4-carboxamide; 4-(2-(2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxamido)ethyl)phenyl (2-((2-((tert-butoxycarbonyl)(methyl)amino)ethyl)disulfaneyl)ethyl)(methyl)carbamate; 4-(2-(2-((S)-1-((2R,3R)-3-((S)-1-((3R,4R,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)thiazole-4-carboxamido)ethyl)phenyl (50-(2,5-dioxo-2,5-dihydro-1H-pyrrol-1-yl)-7-methyl-8,48-dioxo-11,14,17,20,23,26,29,32,35,38,41,44-dodecaoxa-3,4-dithia-7,47-diazapentacontyl)(methyl)carbamate; N2-(4-(((2-amino-4-oxo-4,8-dihydropteridin-6-yl)methyl)amino)benzoyl)-N5-((2S)-4-carboxy-1-((2-((1-(1-(4-(2-(2-((S)-1-((2R,3R)-3-((S)-1-((3R,4R,5S)-4-((S)-2-((S)-2-(dimethylamino)-3-methylbutanamido)-N,3-dimethylbutanamido)-3-methoxy-5-methylheptanoy)pyrrolidin-2-yl)-3-methoxy-2-methylpropanamido)-2-phenylethyl)thiazole-4-carboxamido)ethyl)phenoxy)-2,9-dimethyl-1,10,50-trioxo-13,16,19,22,25,28,31,34,37,40,43,46-dodecaoxa-5,6-dithia-2,9,49-triazadopentacontan-52-yl)-2,5-dioxopyrrolidin-3-yl)thio)ethyl)amino)-1-oxobutan-2-yl)-L-glutamine; N2-(4-(((2-amino-4-oxo-4,8-dihydropteridin-6-yl)methyl)amino)benzoyl)-N5-((2S)-4-carboxy-1-((2-((1-(1-(4-(2-(2-((S)-1-((2R,3R)-3-((S)-1-((3R,4R,5 S)-4-((S)-2-((S)-2-(dimethylamino)-3-methylbutanamido)-N,3-dimethylbutanamido)-3-methoxy-5-methylheptanoy)pyrrolidin-2-yl)-3-methoxy-2-methylpropanamido)-2-phenylethyl)thiazole-4-carboxamido)ethyl)phenoxy)-2,9-dimethyl-1,10,50-trioxo-13,16,19,22,25,28,31,34,37,40,43,46-dodecaoxa-5,6-dithia-2,9,49-triazadopentacontan-52-yl)-2,5-dioxopyrrolidin-3-yl)thio)ethyl)amino)-1-oxobutan-2-yl)-L-glutamine; tert-butyl (2-((2-(2-((S)-1-((2R,3R)-3-((S)-1-((3R,4S,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-methylthiazole-4-carboxamido)ethyl)disulfaneyl)ethyl)(methyl)carbamate; 2-((S)-1-((2R,3R)-3-((S)-1-((3R,4R,5S)-4-((S)-2-((S)-2-(dimethylamino)-3-methylbutanamido)-N,3-dimethylbutanamido)-3-methoxy-5-methylheptanoyl)pyrrolidin-2-yl)-3-methoxy-2-methylpropanamido)-2-phenylethyl)-N-(50-(2,5-dioxo-2,5-dihydro-1H-pyrrol-1-yl)-7-methyl-8,48-dioxo-11,14,17,20,23,26,29,32,35,38,41,44-dodecaoxa-3,4-dithia-7,47-diazapentacontyl)-N-methylthiazole-4-carboxamide; and N2-(4-(((2-amino-4-oxo-4,8-dihydropteridin-6-yl)methyl)amino)benzoyl)-N5-((2S)-4-carboxy-1-((2-((1-(1-(2-((S)-1-((2R,3R)-3-((S)-1-((3R,4R,5S)-4-((S)-2-((S)-2-(dimethylamino)-3-methylbutanamido)-N,3-dimethylbutanamido)-3-methoxy-5-methylheptanoy)pyrrolidin-2-yl)-3-methoxy-2-methylpropanamido)-2-phenylethyl)thiazol-4-yl)-2,9-dimethyl-1,10,50-trioxo-13,16,19,22,25,28,31,34,37,40,43,46-dodecaoxa-5,6-dithia-2,9,49-triazadopentacontan-52-yl)-2,5-dioxopyrrolidin-3-yl)thio)ethyl)amino)-1-oxobutan-2-yl)-L-glutamine.

The dolastatin 10 derivative preferably used in the present invention further includes Demethyl dolastatin 10.

### <Conjugate of anti-CAPRIN-1 antibody and dolastatin 10 or derivative thereof>

In the present invention, the mode of binding between the anti-CAPRIN-1 antibody and the dolastatin 10 or the derivative thereof in the conjugate of the anti-CAPRIN-1 antibody and the dolastatin 10 or the derivative thereof is not particularly limited as long as the antitumor activity against a cancer can be maintained, and preferably is a mode of binding in which a linker structure is formed between the anti-CAPRIN-1 antibody and the dolastatin 10 or the derivative thereof.

In this context, the linker means a compound capable of linking the anti-CAPRIN-1 antibody to the dolastatin 10 or the derivative thereof. Any of various linkers known in the art may be used, or an appropriate chemical modification to the structure of the activating factor may be used for the directly binding.

The details of the type of the linker and the binding method can be basically in accordance with a method known in the art (see, for example, Greg T. Hermanson Bioconjugate Techniques, Third Edition, WO2004/010957, and WO2014/012479).

In an embodiment of the present invention, examples of reactive groups attached to the anti-CAPRIN-1 antibody, the dolastatin 10 or the derivative thereof, and the linker include the following.

Examples of the reactive group attached to the amino acid sequence of the antibody or a glycoprotein modifying an amino acid include primary amine (ε-amino), carboxyl, thiol (sulfhydryl), carbonyl (ketone or aldehyde), and hydroxyl unless a special chemical modification is introduced. The primary amine exists at the N-terminus of a polypeptide or the side chain of a lysine residue and is positively charged under physiological conditions. The primary amine usually exists outside of the protein and can therefore be used in binding without denaturing the structure of the protein. The carboxyl exists at the C-terminus of a polypeptide or the side chain of aspartic acid or glutamic acid. The sulfhydryl exists at the side chain of cysteine and forms a disulfide bond that maintains the higher-order structure of the protein. The ketone or aldehyde is generated in a glycoprotein by the oxidation of glycosyl with sodium metaperiodate.

The conjugate of the present invention is prepared by binding the dolastatin 10 or the derivative thereof to the linker bound to the reactive group of the antibody, binding the dolastatin 10 or the derivative thereof to the linker bound to the dolastatin 10 or the derivative thereof, or directly binding the dolastatin 10 or the derivative thereof to the antibody.

Examples of the reactive groups attached to the linker and the microtubule modulator include the following.

Reactive group capable of reacting with the amine: N-hydroxysuccinimide (NHS) ester, imide ester, pentafluorophenyl ester, hydroxymethyl phosphine, isothiocyanate, isocyanate, acyl azide, N-hydroxyl ester, sulfonyl chloride, aldehyde, glyoxal, epoxide, oxirane, carbonate, aryl, carbodiimide, and carboxylic anhydride.

Reactive group capable of reacting with the carboxyl and the amine: carbodiimide, diazoalkane, diazoacetyl compounds, and carbonyldiimidazole.

Reactive group capable of reacting with the thiol: maleimide, haloacetamide, pyridyl disulfide, thiosulfone, vinyl sulfone, haloacetyl, aziridine, acryloyl, and aryl.

Reactive group capable of reacting with the aldehyde: hydrazide and alkoxyamine. Reactive group capable of reacting with the hydroxyl: epoxy, oxirane, carbonyldiimidazole, N,N'-disuccinimidyl carbonate, N-hydroxysuccinimidyl chloroformate, and isocyanate.

Reactive group capable of reacting with the hydroxyl: isocyanate.

Photoreactive reactive group: diaziridine, aryl azide, aryl, benzophenol, and diazo compounds.

Specific examples of the linker having said reactive group include the following.

As a linker having the same reactive group ends, a linker having N-hydroxysuccinimide ester as a reactive group (e.g., Disuccinimidyl Glutarate (DSG), disuccinimidyl suberate (DSS), bis(sulfosuccinimidyl)suberate (BS3), tris-(succinimidyl)aminotriacetate (TSAT), PEGylated bis(sulfosuccinimidyl)suberate (BS(PEG)s, BS(PEG)₉), dithiobis (succinimidyl propionate) (DSP), 3,3'-dithiobis (sulfosuccinimidyl propionate) (DTSSP), ethylene glycol bis (succinimidyl succinate) (EGS), Sulfo-ethylene glycol bis (succinimidyl succinate (Sulfo-EGS), Dimethyl adipimidate·2HCl (DMA), Dimethyl pimelimidate·2HCl (DMP), Dimethyl suberimidate·2HCl (DMS), Dimethyl3,3'-dithiobispropionimidate·2HCl (DTBP), 1,5-difluoro-2,4-dinitrobenzene (DFDNB), Disuccinimidyl tartrate (DST), Bis[2-(Succinimidooxycarbonyloxy)ethyl]Sulfone (BSOCOES) and a linker having maleimide as a reactive group (e.g., Bismaleimidoethane (BMOE), 1,4-bismaleimidobutane (BMB), Bismaleimidohexane (BNM), Tris(2-maleimidothyl)amine (TMEA), 1,8-bismaleimido-(PEG)₂ (BM(PEG)₂), 1,8-bismaleimido-(PEG)₃(BM(PEG)₃), and Dithiobismaleimidoethane (DTME)).

As a linker having different reactive group ends, a linker having NHS ester and maleimide as reactive groups (e.g., AMAS, BMPS, GMBS, Sulfo-MBS, MBS, Sulfo-MBS, SMCC, Sulfo-SMCC, EMCS, Sulfo-EMCS, SMPB, Sulfo-SMPB, SMPH, LC-SMCC, Sulfo-KMUS, SM(PEG)₂, SM(PEG)₄, SM(PEG)₆, SM(PEG)₈, SM(PEG)₁₂, and SM(PEG)₂₄), a linker having NHS ester and pyridyldithiol as reactive groups (e.g., SPDP, LC-SPDP, Sulfo-LC-SPDP, SMPT, (PEG)₄-SPDP, and PEG12-SPDP), a linker having NHS ester and haloacetyl as reactive groups (e.g., SIA, SBAP, SIAB, and Sulfo-SIAB), a linker having NHS ester and aryl azide as reactive groups (e.g., ANB-NOS, Sulfo-SANPAH, and ATFB), a linker having NHS ester and diaziridine as reactive groups (e.g., SDA, Sulfo-SDA, LC-SDA, SDAD, and Sulfo-SDAD), a linker having carbodiimide as reactive groups (e.g., DCC, EDC, EDAC, NHS, and Sulfo-NHS), a linker having maleimide and hydrazide as reactive groups (e.g., BMPH, EMCH, MPBH, and KMUH), a linker having pyridyldithiol and hydrazide as a reactive group (e.g., PDPH), a linker having isocyanate and maleimide as reactive groups (e.g., PMPI), and a linker having NHS ester and psoralen as reactive groups (e.g., SPB).

As other linkers, a linker containing a polypeptide, for example, Fmoc-Ala-Ala-Asn-PAB, Fmoc-Ala-Ala-Asn(Trt)-PAB, Fmoc-PEG₃-Ala-Ala-Asn(Trt)-PAB, Fmoc-PEG₄-Ala-Ala-Asn(Trt)-PAB, Fmoc-Ala-Ala-Asn-PAB-PNP, Fmoc-Ala-Ala-Asn(Trt)-PAB-PNP, Fmoc-PEG₃-Ala-Ala-Asn(Trt)-PAB-PNP, Azide-PEG₄-Ala-Ala-Asn(Trt)-PAB-PNP, Mal-PEG₄-Ala-Ala-Asn(Trt)-PAB-PNP, Fmoc-Val-Cit-PAB-OH, Val-Cit-PAB-OH, Fmoc-Val-Cit-PAB-PNP, MC-Val-Cit-PAB, MC-Val-Cit-PAB-PNP, Phe-Lys(Trt)-PAB, Fmoc-Phe-Lys(Trt)-PAB-PNP, Fmoc-Gly3-Val-Cit-PAB, Fmoc-Gly3-Val-Cit-PAB-PNP, Ala-Ala-Asn-PAB TFA salt.

In addition, Bis-PEG-acid, PEG Acid (e.g., Acid-PEG-TEMPO, Amino-PEG-acid, Amino-PEG-CH₂CO₂H, Aminoxy-PEG-acid, Azido-PEG-acid, Carboxy-PEG-sulfonic acid, Fmoc-N-amido-PEG-acid, Fmoc-N-amido-PEG-CH₂CO₂H, Fmoc-aminooxy-PEG-acid, Hydroxy-PEG-acid, Hydroxy-PEG-CH₂CO₂H, m-PEG-acid, m-PEG-(CH₂)₃-acid, Methoxytrityl-N-PEG-acid, N-methyl-N-(t-Boc)-PEG-acid, Propargyl-PEG-acid, Propargyl-PEG-CH₂CO₂H, Propargyl-PEG-(CH₂)₃-acid, t-Boc-N-amido-PEG-acid, t-Boc-N-amido-PEG-CH₂CO₂H, t-Boc-Aminooxy-PEG-acid, Acid-PEG-PFP ester, Miscellaneous PEG acid), PEG PFP ester (e.g., Acid-PEG-PFP ester, Bis-PEG-PFP ester), Bis-PEG-NHS, PEG Aldehyde (e.g., m-PEG-aldehyde, m-PEG-benzaldehyde, Ald-PEG-acid, Ald-PEG-amine, Ald-PEG-azide, Ald-PEG-NH-Boc, Ald-PEG-NHS ester, Ald-PEG-TFP ester, Ald-PEG-t-butyl ester), PEG Tosylate (e.g., Azido-PEG-Tos, Hydroxy-PEG-Tos, m-PEG-Tos, t-Boc-Aminooxy-PEG-Tos, Trifluoroethyl-PEG-Tos, Tos-PEG-acid, Tos-PEG-CH₂CO₂H, Tos-PEG-alkyne, Tos-PEG-t-butyl ester, Tos-PEG-CH₂CO₂tBu, Tos-PEG-Tos, S-acetyl-PEG6-Tos, N-Tos-N-(t-butoxycarbonyl)-aminooxy-PEG₄-Tos, Ms-PEG-Ms, Ms-PEG-t-butyl ester, PEG-Ms, Propargyl-PEG-Ms), Boc-PEG (e.g., Amino-PEG-t-Boc-Hydrazide, Azido-PEG-t-Boc-Hydrazide, Boc-NH-PEG-NH-Boc, Bromoacetamido-PEG-Boc-amine, m-PEG-ONHBoc, Mal-Alkyl-t-Boc-amine, N-Boc-PEG-alcohol, N-Boc-PEG-bromide, N-methyl-N-(t-Boc)-PEG-acid, t-Boc-N-amido-PEG-acid, t-Boc-N-amido-PEG-CH₂CO₂H, t-Boc-N-Amido-PEG-amine, t-Boc-N-amido-PEG-azide, t-Boc-N-amido-PEG-NHS ester, t-Boc-N-amido-PEG-sulfonic acid), PEG NHS ester (e.g., Acid-PEG-NHS ester, Azido-PEG-NHS ester, Bis-PEG-NHS, Fmoc-PEG-NHS ester, m-PEG-NHS ester, m-PEG-NHS Carbonate, Mal-PEG-NHS ester, Propargyl-PEG-NHS ester, t-Boc-N-amido-PEG-NHS ester, t-Butoxycarbonyl-PEG-NHS ester), Fmoc-PEG (e.g., Fmoc-N-amido-PEG-acid, Fmoc-NH-PEG-CH₂CO₂H, Fmoc-PEG-NHS ester), Biotin PEG (e.g., Biotin PEG-acid, Biotin PEG-alcohol, Biotin PEG-alkyne, Biotin PEG-amine, Biotin PEG-azide, Biotin PEG-DBCO, Biotin PEG-hydrazide, Biotin-PEG-Mal, Biotin-PEG-NHS, Biotin-EDA-PEG-NHS, Biotin-PEG-oxyamine, Biotin-PEG-PFP, Biotin-EDA-PEG-PFP, Biotin-PEG-Tetrazine, Biotin-PEG-TFP, Azide-SS-biotin, Biotin-PEG3-SS-azide, DBCO-S-S-PEG3-Biotin, Dde Biotin-PEG4-Alkyne, Dde Biotin-PEG₄-Azide, Dde Biotin-PEG₄-DBCO, Diazo Biotin-PEG₃-Alkyne, Diazo Biotin-PEG₃-Azide, Diazo Biotin-PEG3-DBCO, Diol Biotin-PEG₃-Alkyne, Diol Biotin-PEG₃-Azide, PC Biotin-PEG₃-Alkyne, PC-Biotin-PEG₄-PEG₄-Alkyne, PC-Biotin-PEG₄-PEG4-Alkyne, PC Biotin-PEG₃-Azide, PC-Biotin-PEG4-PEG3-Azide, PC-Biotin-PEG₄-NHS carbonate, PC DBCO-PEG₃-Biotin, WSPC Biotin-PEG₃-DBCO, Fmoc-Lys (biotin-PEG)-OH, Fmoc-N-amido-(PEG-biotin)-acid, TAMRA-Azide-PEG-Biotin), PEG Phosphonate, Aminooxy PEG (e.g., Aminooxy-PEG-acid, Aminooxy-PEG-alcohol, Aminooxy-PEG-azide, Aminooxy-PEG-bromide, Aminooxy-PEG-methane, Aminooxy-PEG-Propargyl, Aminooxy-PEG-t-butyl ester, Aminooxy-PEG-Thiol, Bis-(Aminooxy)-PEG, t-Boc-Aminooxy-PEG-acid, t-Boc-Aminooxy-PEG-alcohol, t-Boc-Aminooxy-PEG-amine, t-Boc-Aminooxy-PEG-Azide, t-Boc-Aminooxy-PEG-Bromide, t-Boc-aminooxy-PEG-Methane, t-Boc-aminooxy-PEG-Propargyl, t-Boc-aminooxy-PEG-S-Ac, t-Boc-Aminooxy-PEG-Thiol, t-Boc-Aminooxy-PEG-Tos, Fmoc-aminooxy-PEG-acid, Trifluoroethyl-PEG-Aminooxy), Alkyne PEG (e.g., endo-BCN-PEG, exo-BCN-PEG, Propargyl-PEG-acid, Propargyl-PEG-CH₂CO₂H, Propargyl-PEG-(CH₂)₃-acid, Propargyl-PEG-(CH₂)₃-methyl ester, Propargyl-PEG-Acrylate, Propargyl-PEG-alcohol, Propargyl-PEG-amine, Propargyl-PEG-methylamine, Aminooxy-PEG-Propargyl, Propargyl-PEG-azide, Propargyl-PEG-bromide, Propargyl-PEG-Maleimide, Propargyl-PEG-Ms, Propargyl-PEG-NHS ester, Propargyl-PEG-sulfonic acid, Propargyl-PEG-t-butyl ester, Propargyl-PEG-CH₂CO₂tBu, Propargyl-PEG-thiol, Propargyl-PEG-5-nitrophenyl carbonate, t-Boc-aminooxy-PEG-Propargyl, Bis-Propargyl-PEG, m-PEG-Propargyl), Azido PEG (e.g., Azido-PEG-acid, Azido-PEG-CH₂CO₂H, Azido-PEG-(CH₂)₃-methyl ester, Azido-PEG-Acrylate, Azido-PEG-alcohol, Azido-PEG-(CH₂)₃OH, Azido-PEG-amine, Azido-PEG-azide, Azido-PEG-Maleimide, Azido-PEG-methylamine, Azido-PEG-methyl ester, Azido-PEG-NHS ester, Azido-PEG-CH₂CO₂-NHS, Azido-PEG-oxazolidin-2-one, Azido-PEG-PFP ester, Azido-PEG-phosphonic acid, Azido-PEG-phosphonic acid ethyl ester, Azido-PEG-sulfonic acid, Azido-PEG-t-Boc-Hydrazide, Azido-PEG-t-butyl ester, Azido-PEG-CH₂CO₂-t-butyl ester, Azido-PEG-TFP ester, Azido-PEG-Tos, Aminooxy-PEG-azide, Bromo-PEG-azide, Bromoacetamido-PEG-azide, Carboxyrhodamine 110-PEG-Azide, Isothiocyanato-PEG-Azide, Isothiocyanato-PEG-Azide, m-PEG-azide, Propargyl-PEG-azide, TAMRA-PEG-Azide, t-Boc-N-Amido-PEG-Azide, t-Boc-Aminooxy-PEG-Azide, Thiol-PEG-Azide, Trifluoroethyl-PEG-Azide, Azido-PEG-amino acid, Azido-PEG₄-4-nitrophenyl carbonate, S-Acetyl-PEG₃-Azido, Azide, Trityl-PEG₁₀-Azide), Alkyne PEG, DBCO-PEG, BCN-PEG, Propargyl-PEG, Bis-PEG-acid, Bis-PEG-NHS, Bis-PEG-PFP, Bis-Propargyl-PEG, Amine-PEG-Amine, Azido-PEG-azide, Bromo-PEG, or Mal PEG may be used.

Furthermore, Py-ds-Prp-Osu, Py-ds-dmBut-OSu, Py-ds-dmBut-OPFP, Py-ds-Prp-OPFP, MAL-HA-OSu, MAL-di-EG-OPFP, MAL-tri-EG-OPFP, MAL-tetra-EG-OPFP, N3-di-EG-OPFP, N3-tri-EG-OPFP, N3-tetra-EG-OPFP, ALD-BZ-OSu, ALD-di-EG-OSu, ALD-tetra-EG-OSu, ALD-di-EG-OPFP, ALD-tetra-EG-OPFP, PHA-di-EG-OPFP, PHA-tetra-EG-OPFP may be used.

In another embodiment, polyethylene glycols (PEGs) described in WO2015/057699 and WO2017/165851 may be used to obtain conjugates that are expected to provide better *in vivo* kinetics.

In another embodiment, the linker described in US10808039 or a polypeptide represented by -Gly-Gly-Phe-Gly- may be used.

The linker between the anti-CAPRIN-1 antibody and the dolastatin 10 or the derivative thereof may be composed of a single type or composed of a plurality of types.

A method for preparing the conjugate of the anti-CAPRIN-1 antibody and the dolastatin 10 or the derivative thereof includes a method which involves binding the dolastatin 10 or the derivative thereof using a ε-amino group at the lysine side chain of the antibody, and a method which involves binding using thiol formed by the reduction treatment of a cysteine residue constituting the disulfide bond of the antibody.

In the case of using a ε-amino group at the lysine residue of the antibody, for example, a method is used which involves reacting active ester (e.g., N-hydroxysuccinimide ester) therewith to form an amide bond. In this case, since the antibody contains many lysine residues, the binding reaction proceeds nonspecifically. In the present embodiment, for example, sc-vc-PAB-MMAE or Osu-Glu-vc-PAB-MMAE may be used.

In the case of using thiol constituting a disulfide bond and present at the side chain of cysteine in the antibody, a method in which thiol is formed from the disulfide bond on the antibody using a reducing agent such as mercaptoethanol and reacted with maleimide or α-haloamide is used. For example, a method using sulfone phenyloxadiazole, a 4-cyanoethynyloxy derivative, or the like is used for stabilizing a thiol-mediated bond. These bonds are stable for a longer time than the bond based on the conjugate addition reaction of cysteine with maleimide. In addition, a linker having an amino group near an imide group may also be used because an imide ring by adding a thiol group to maleimide is opened by hydrolysis so that stability is improved owing to the resulting amide bond. The thiols of cysteines form a disulfide bond in the antibody, and thus an alternative method involves binding the dolastatin 10 or the derivative thereof via two thiols by binding in between. As an example, a crosslinked bond may be formed using a linker having two disulfide bond sites that can be formed from an amide group having two sulfones at the β position, or dibromomaleimide.

The conjugate of the present invention may be obtained by a method using, for example, the THIOMAB^{™} technique or ThioBridge^{™}, which is a method capable of introducing a determined number of thiol groups at a particular site of an antibody (see Nature Biotechnology 26, 925-932 (2008) or Biocojugate Chem., 25(6), 1124-1136 (2014)).

The conjugate of the present invention may be formed, for example, by reducing the antibody using a reducing agent dithiothreitol (DTT) in a phosphate buffer to obtain an antibody having a reactive thiol group, which is then conjugated with the dolastatin 10 or the derivative thereof. The conjugate can be obtained by adding a thiol group to primary amine at the lysine residue of the antibody by the introduction of a Traut's reagent (2-iminothiolane or N-succinimidyl S-acetylthioacetate (SATA)), instead of the method using a reducing agent.

The amount of the thiol added to the antibody can be determined, for example, by mixing a sample solution containing 5,5'-dithiobis(2-nitrobenzoic acid) (DTNB) and an SH group with a phosphate buffer solution (pH 8.0) and distilled water, adding a solution of DTNB dissolved in a phosphate buffer, a Good's buffer, or a Tris buffer to the mixture, and incubating the resulting for a given time, followed by the measurement of absorbance at 412 nm (see G.L. Ellman, Arch. Biochem. Biophys., 82, 70 (1959)).

The thiol group added by the cleavage of the disulfide bond of the antibody through reduction treatment is preferably treated (capped) in order to prevent the formation of a disulfide bond again. For example, N-ethylmaleimide (NEM) or 2-iodoacetamide (IAA) may be employed for the capping.

The conjugate can be constructed through binding the dolastatin 10 or the derivative thereof to the antibody using the thiol group added to the antibody according to a method known in the art. Specifically, the binding can be carried out using, for example, a linker reagent having a maleimide group or a bromoacetamide group as a linker reagent specifically binding to the thiol group of the reduced antibody. For example, N-succinimidyl-4-(N-maleimidomethyl)-cyclohexane-1-carboxylate (SMCC) is used as the linker having a maleimide group. In this case, the N-succinimide group of SMCC can form an amide bond with an amino group present in the dolastatin 10 or the derivative thereof to obtain the conjugate.

In another embodiment, an amide bond is first formed at an amino group present in the activating factor using SMCC. Then, the maleimide group of the SMCC bound with the dolastatin 10 or the derivative thereof can be reacted with the thiol group added to the antibody to obtain the conjugate.

In another embodiment, the conjugate of the antibody and the dolastatin 10 or the derivative thereof may be formed using two linkers. For example, a primary amino group present at the lysine residue of the antibody is bound to the N-succinimide group of SATA (N-succinimidyl-S-acetylthioacetate) via an amide bond to add a thiol group to the antibody. SMCC is reacted with the dolastatin 10 or the derivative thereof containing an amino group or with the dolastatin 10 or the derivative thereof having an amino group added thereto according to an ordinary method to form an amide bond with the N-succinimide group of the SMCC. Then, the maleimide group of the SMCC bound with the dolastatin 10 or the derivative thereof can be reacted with the thiol group of the SATA bound with the antibody to obtain the conjugate.

In another further embodiment, examples of the preparation of the conjugate of the antibody and the dolastatin 10 or the derivative thereof include a method using maleimidocaproyl-valine-citrulline-p-aminobenzyloxycarbonyl (MC-Val-Cit-PAB) as a linker. MC-val-Cit-PAB (mc-vc-PAB) is a linker cleavable by intracellular protease (e.g., cathepsin B). A thiol group is added to the antibody dissolved in a phosphate buffer using DTT or the like. Meanwhile, the dolastatin or the dolastatin derivative having an amino group is reacted with the benzyloxycarbonyl (PAB) in MC-Val-Cit-PAB to prepare dolastatin 10 or a derivative thereof bound with MC-val-Cit-PAB, which can then be reacted with the thiol-added antibody described above to obtain the conjugate. For example, the conjugate is mc-val-Cit-PAB-MMAE in which MMAE, one of the dolastatin derivatives, is linked to mc-val-Cit-PAB by a predetermined method, Vedotin to which MMAE is linked, or mafadotin to which MMAF is linked.

In another further embodiment, the conjugate is included in which SATA is bound to a primary amino group at the lysine residue of the antibody to add a thiol group thereto, and succinimidyl 3-(2-pyridyldithio)propionate (SPDP) is reacted with the dolastatin 10 or the derivative thereof having an amino group to form an amide bond with the N-succinimide group of the SPDP.

The linker used in the present invention is cleavable under intracellular conditions, and a substance having antitumor activity, which contains dolastatin 10, a derivative thereof, or a part of dolastatin 10 or a derivative thereof and the linker is released in a cell. For example, the linker is a linker cleaved by peptidase or protease in a cell. Preferably, the linker is a linker cleaved by lysosome, endosomal protease, cathepsin B, cathepsin D, or plasmin. Examples thereof include a linker comprising a polypeptide (Val-Cit, Phe-Leu, or Gly-Phe-Leu-Gly) cleavable by cathepsin B. More specifically, the linker described in U.S. Patent No. 6,214,345 may be used.

In another further embodiment, for example, a linker having glucuronic acid (preferably β-D-glucuronide) described in WO2007/0711968 may be used in order to improve the stability, solubility, and metabolism in blood of the conjugates of the present invention and the binding capacity of dolastatin 10 or a derivative thereof to the anti-CAPRIN-1 antibody. Alternatively, the methods described in WO2013/173337, WO2015/095755, WO2015/123679, and WO2018/031690 may be used.

In another further embodiment, for example, methods described in WO2006/65533 and WO2018/160683 may be used to site-specifically bind dolastatin 10 or dolastatin 10 derivatives to anti-CAPRIN-1 antibodies.

In another further embodiment, conjugates of two or more drugs including dolastatin 10 or a dolastatin 10 derivative with anti-CAPRIN-1 antibodies may be prepared by the method described in WO2018/112253. Preferably, one of the two or more drugs is MMAE or MMAF.

In order to obtain a composition comprising the conjugate of the anti-CAPRIN-1 antibody and the dolastatin 10 or the derivative thereof of the present invention, for example, a peak fraction of a higher molecular weight compared to the antibody before the binding of the linker may be collected by the application of gel filtration chromatography or the like. The method described in, for example, WO2013/049410 may be used to detect the mass of the conjugate while maintaining an intact bivalent antibody.

The number of molecules of the bound dolastatin 10 or a derivative thereof per antibody molecule in the conjugate of the anti-CAPRIN-1 antibody and the dolastatin 10 or the derivative thereof of the present invention may be quantified according to known chromatographic methods such as mass spectrometry, ELISA, electrophoresis, and HPLC.

### <Antitumor effect of conjugate>

The conjugate of the anti-CAPRIN-1 antibody and the dolastatin 10 or the derivative thereof of the present invention has antitumor activity *in vitro* or *in vivo.* The antitumor activity means reduction, elimination, growth inhibition, apoptosis, necrosis, or killing of target cancer cells. Accordingly, the antitumor effect of the conjugate of the present invention may be determined by examining its antitumor activity against a cancer.

In order to determine the antitumor effect *in vivo,* the antitumor activity can be evaluated by: administering the conjugate to an organism having a cancer; and examining the size of the cancer over time via measuring the size of the tumor after the administration. The antitumor effect of the present invention can also be evaluated by examining a survival rate. Alternatively, the antitumor effect of the present invention may be evaluated by examining the ability to produce a cytokine or a chemokine. The antitumor effect of the conjugate of the present invention can be further determined by examining the prevention of a cancer, the prevention of metastasis, or the prevention of recurrence.

The conjugate of the present invention is expected to have a stronger antitumor effect, when the conjugate has higher binding affinity for the CAPRIN-1 protein on cancer cell surface. Its association constant (affinity constant) Ka (kon/koff) is preferably at least 10⁷ M⁻¹, at least 10⁸ M⁻¹, at least 5 x 10⁸ M⁻¹, at least 10⁹ M⁻¹, at least 5 x 10⁹ M⁻¹, at least 10¹⁰ M⁻¹, at least 5 x 10¹⁰ M⁻¹, at least 10¹¹ M⁻¹, at least 5 x 10¹¹ M⁻¹, at least 10¹² M⁻¹, or at least 10¹³ M⁻¹.

The ability of the conjugate of the present invention to bind to CAPRIN-1 can be identified through the use of binding assay using, for example, surface plasmon resonance (SPR), ELISA, Western blot, immunofluorescence, or flow cytometry.

The conjugate of the present invention enhances the antitumor effect compared to the anti-CAPRIN-1 antibody alone, as described above. The rate of the enhancement is preferably 30% or more, more preferably 40% or more, further preferably 50% or more, still further preferably 55% or more, even further preferably 60% or more, furthermore preferably 65% or more, most preferably 70% or more. The rate of enhancement in antitumor effect by the conjugate of the present invention with respect to the anti-CAPRIN-1 antibody alone can be calculated by administering their respective effective amounts to cancer-bearing mice under the same conditions, and comparing tumor volumes 10 days or later after the start of the administration.

### <Pharmaceutical composition, method for treating and/or preventing cancer>

The target of the pharmaceutical composition for the treatment and/or prevention of a cancer of the present invention is not particularly limited as long as the target is cancer (cells) expressing the CAPRIN-1 protein.

The terms "tumor" and "cancer" used in the present specification mean malignant neoplasm and are used interchangeably with each other.

The cancer targeted in the present invention may be any cancer expressing the CAPRIN-1 protein on the cell membrane surface. The cancer is preferably breast cancer, kidney cancer, pancreatic cancer, colon cancer, lung cancer, brain tumor, gastric cancer, uterine cancer, ovarian cancer, prostate cancer, bladder cancer, esophageal cancer, leukemia, lymphoma, liver cancer, gallbladder cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, urothelial carcinoma, renal cell carcinoma, colorectal cancer, gastroesophageal junction cancer, hepatocellular carcinoma, glioblastoma, primary central nervous system lymphoma, primary testicular lymphoma, biliary tract cancer, sarcoma, fibrosarcoma, basal cell carcinoma, Paget's disease, or skin cancer as described above.

More specifically, examples of these cancers include breast adenocarcinoma, composite type breast adenocarcinoma, malignant mammary mixed tumor, intraductal papillary adenocarcinoma, recurrent metastatic breast cancer, lung adenocarcinoma, non-small cell lung cancer (NSCLC), squamous non-small cell lung cancer, squamous cell cancer, small-cell cancer, large-cell cancer, glioma that is a tumor of neuroepithelial tissue, glioblastoma, neuroblastoma, ependymoma, neuronal tumor, embryonal neuroectodermal tumor, schwannoma, neurofibroma, meningioma, chronic lymphocytic leukemia, lymphoma, gastrointestinal lymphoma, digestive lymphoma, small-cell-to-medium-cell lymphoma, cecal cancer, ascending colon cancer, descending colon cancer, transverse colon cancer, sigmoid colon cancer, rectal cancer, epithelial ovarian cancer, germ cell tumor, stromal cell tumor, pancreatic ductal carcinoma, invasive pancreatic ductal carcinoma, pancreatic adenocarcinoma, metastatic adenocarcinoma, acinar cell carcinoma, adenosquamous carcinoma, giant cell tumor, intraductal papillary-mucinous neoplasm, mucinous adenocarcinoma, pancreatoblastoma, islet-cell adenoma, Frantz tumor, serous cystadenocarcinoma, solid-pseudopapillary cancer, gastrinoma, glucagonoma, insulinoma, multiple endocrine neoplasia type-1 (Wermer's syndrome), nonfunctional islet cell tumor, somatostatinoma, VIPoma, uterine cervix cancer, uterine body cancer, fibrosarcoma, sarcoma of bones or joints, Ewing's sarcoma, Wilms tumor, hepatoblastoma, soft tissue sarcoma, acute leukemia, chronic leukemia, spinal cord tumor, malignant soft tissue tumor, teratoma group tumor, and head and neck cancer including hypopharynx cancer, oropharynx cancer, tongue cancer, nasopharyngeal cancer, oral cavity cancer, lip cancer, sinus cancer, laryngeal cancer, and recurrent brain tumor. Further examples thereof include, but are not limited to, renal pelvis/urinary tract cancer, bladder cancer, urethral cancer, testicular tumors, malignant pleural mesothelioma, malignant osteosarcoma, and pediatric malignant solid tumors (rhabdomyosarcomas, neuroblastomas, hepatoblastomas, medulloblastomas, nephroblastoma, retinoblastomas, central nervous system germ cell tumors, and Ewing' sarcoma family of tumors).

The subjects (patients) to be targeted are preferably mammals, for example, mammals including primates, pet animals, livestock, and sport animals and are particularly preferably humans, dogs, and cats.

In the case of using the conjugate used in the present invention in a pharmaceutical composition, the pharmaceutical composition can be formulated by a method known to those skilled in the art. For example, the pharmaceutical composition may be used in the form of a parenterally injectable agent of an aseptic solution or suspension with water or any other pharmaceutically acceptable liquid. For example, the conjugate may be formulated in a unit dosage form required for generally accepted pharmaceutical practice, by mixing with pharmacologically acceptable carriers or media, specifically, sterilized water, physiological saline, a plant oil, an emulsifier, a suspending agent, a surfactant, a stabilizer, a fragrance, an excipient, a binder, or the like, in appropriate combination. The amount of the active ingredient in such a preparation is determined so that an appropriate dose within the prescribed range can be achieved.

In the case of using the conjugate of the present invention in a pharmaceutical composition, the pharmaceutical composition may be formulated in a lyophilized state, comprising any salts, surfactants, buffers, sugars, cryoprotectants (including some sugars).

An aseptic composition for injection can be formulated according to conventional pharmaceutical practice using a vehicle such as injectable distilled water. Examples of aqueous solutions for injection include saline, isotonic solutions containing glucose and other auxiliary agents, for example, D-sorbitol, D-mannose, D-mannitol, and sodium chloride. These solutions may be used in combination with an appropriate solubilizer, for example, an alcohol (specifically, ethanol) or a polyalcohol (e.g., propylene glycol and polyethylene glycol), or a nonionic surfactant, for example, Polysorbate 80^{™} or HCO-60. Examples of oil solutions include sesame oil and soybean oil, and may be used in combination with benzyl benzoate or benzyl alcohol as a solubilizer. The solutions may also be mixed with a buffer (e.g., a phosphate buffer solution and a sodium acetate buffer solution), a soothing agent (e.g., procaine hydrochloride), a stabilizer (e.g., benzyl alcohol and phenol), and an antioxidant. The injection solutions thus prepared are usually filled into appropriate ampules. Examples of oil solutions include sesame oil and soybean oil, and may be used in combination with benzyl benzoate or benzyl alcohol as a solubilizer. The solutions may also be mixed with a buffer (e.g., a phosphate buffer solution and a sodium acetate buffer solution), a soothing agent (e.g., procaine hydrochloride), a stabilizer (e.g., benzyl alcohol and phenol), and an antioxidant. The injection solutions thus prepared are usually filled into appropriate ampules.

The administration is carried out orally or parenterally, preferably parenterally. Specific examples include injectable agents, intranasal administration preparations, transpulmonary administration preparations, and percutaneous administration preparations. Examples of the injectable agents include intravenous injection, intramuscular injection, intraperitoneal injection, subcutaneous injection, and intratumoral administration, through which the pharmaceutical composition can be administered systemically or locally.

Also, the administration method may be appropriately selected in view of the age, weight, sex, symptoms, or the like of a patient. The dose of a pharmaceutical composition containing the antibody or a polynucleotide encoding the antibody may be selected within a range of, for example, 0.0001 mg to 1000 mg per kg of body weight per dose, for example, 0.5 mg, 1 mg, 2 mg, 3 mg, 5 mg, 10 mg, 20 mg, 50 mg, 75 mg, 100 mg, 200 mg, 500 mg, or 1000 mg per kg of body weight per dose. Alternatively, the dose can be selected within a range of, for example, 0.001 to 100000 mg/body of a patient, though the dose is not necessarily limited to these numeric values.

Although the dose and the administration method vary depending on the weight, age, sex, symptoms, or the like of a patient, those skilled in the art can appropriately select the dose and the method.

The pharmaceutical composition for the treatment and/or prevention of a cancer comprising the conjugate of the present invention as an active ingredient can be administered to a subject to treat and/or prevent the aforementioned cancer expressing CAPRIN-1 on the cell membrane surface, preferably breast cancer, kidney cancer, pancreatic cancer, colon cancer, lung cancer, brain tumor, gastric cancer, uterine cancer, ovarian cancer, prostate cancer, bladder cancer, esophageal cancer, leukemia, lymphoma, liver cancer, gallbladder cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, urothelial carcinoma, renal cell carcinoma, colorectal cancer, gastroesophageal junction cancer, hepatocellular carcinoma, glioblastoma, primary central nervous system lymphoma, primary testicular lymphoma, biliary tract cancer, sarcoma, fibrosarcoma, basal cell carcinoma, Paget's disease, or skin cancer.

The pharmaceutical composition for the treatment and/or prevention of a cancer comprising the conjugate of the present invention as an active ingredient can be administered with one type or two or more types of antitumor agents together or separately in combination to a subject to treat and/or prevent the cancer. The antitumor agents that may be used in the present invention include alkylating agents, platinum complexes, topoisomerase inhibitors, antimetabolites, anticancer antibiotics, alkaloid antitumor agents, and immune checkpoint inhibitors antitumor agents which inhibit angiogenesis; and molecular target drugs, used in standard cancer treatments.

### Examples

Hereinafter, the present invention will be specifically described with reference to Examples. However, the scope of the present invention is not intended to be limited by these specific examples.

### (Example 1) Anti-CAPRIN-1 polyclonal antibody

In order to obtain anti-CAPRIN-1 polyclonal antibodies having immunological reactivity against the CAPRIN-1 protein to be used in conjugates of the present invention, 1 mg of a recombinant human CAPRIN-1 protein of SEQ ID NO: 2 or SEQ ID NO: 4 produced according to Example 3 of WO2010/016526 was mixed with an equal volume of incomplete Freund's adjuvant (IFA) solution, and this mixture was subcutaneously administered to rabbits four times every 2 weeks. Then, blood was collected to obtain antiserum containing polyclonal antibodies. The obtained antiserum was purified using a protein G carrier (GE Healthcare BioSciences Corp.) to prepare a polyclonal antibody against the CAPRIN-1 protein (anti-CAPRIN-1 polyclonal antibody #1). Additionally, the serum of a rabbit to which an antigen is not administered was purified using a protein G carrier in the same manner as above and used as a rabbit control antibody.

The following polyclonal antibodies #2 to #6 against partial polypeptides of CAPRIN-1 were obtained in the same manner as in the method for preparing the polyclonal antibody against the CAPRIN-1 protein.

Anti-CAPRIN-1 polyclonal antibody #2 against a partial CAPRIN-1 polypeptide represented by SEQ ID NO: 37 disclosed in WO2011/096528 (SEQ ID NO: 31 of the present specification), anti-CAPRIN-1 polyclonal antibody #3 against a partial polypeptide represented by SEQ ID NO: 5 disclosed in WO2013/018894 (SEQ ID NO: 32 of the present specification), anti-CAPRIN-1 polyclonal antibody #4 against a partial polypeptide represented by SEQ ID NO: 5 disclosed in WO2013/125654 (SEQ ID NO: 33 of the present specification), anti-CAPRIN-1 polyclonal antibody #5 against a partial polypeptide represented by SEQ ID NO: 37 disclosed in WO2011/096533 (SEQ ID NO: 34 of the present specification), and anti-CAPRIN-1 polyclonal antibody #6 against a partial polypeptide represented by SEQ ID NO: 37 disclosed in WO2011/096534 (SEQ ID NO: 35 of the present specification).

### (Example 2) Anti-CAPRIN-1 monoclonal antibody

The following anti-CAPRIN-1 monoclonal antibodies were used in the conjugate of the present invention.

The monoclonal antibody against CAPRIN-1 disclosed in WO2011/096528, wherein the antibody comprises CDR1-3 of heavy chain variable region consisting of the amino acid sequences of SEQ ID NO: 36, SEQ ID NO: 37, and SEQ ID NO: 38, respectively, and CDR1-3 of light chain variable region consisting of the amino acid sequences of SEQ ID NO: 40, SEQ ID NO: 41, and SEQ ID NO: 42, respectively (e.g., an antibody comprising the amino acid sequence of a heavy chain variable region represented by SEQ ID NO: 39 comprising said CDR1-3 of heavy chain variable region, and the amino acid sequence of a light chain variable region represented by SEQ ID NO: 43 comprising said CDR1-3 of light chain variable region).

The monoclonal antibody against CAPRIN-1 disclosed in WO2015/020212, wherein the antibody comprises CDR1-3 of heavy chain variable region consisting of the amino acid sequences of SEQ ID NO: 44, SEQ ID NO: 45, and SEQ ID NO: 46, respectively, and CDR1-3 of light chain variable region consisting of the amino acid sequences of SEQ ID NO: 48, SEQ ID NO: 49, and SEQ ID NO: 50, respectively (e.g., an antibody comprising the amino acid sequence of a heavy chain variable region represented by SEQ ID NO: 47 comprising the CDR1-3 of heavy chain variable region, and the amino acid sequence of a light chain variable region represented by SEQ ID NO: 51 comprising the CDR1-3 of light chain variable region).

The monoclonal antibody against CAPRIN-1 disclosed in WO2011/096519, wherein the antibody comprises CDR1-3 of heavy chain variable region consisting of the amino acid sequences of SEQ ID NO: 52, SEQ ID NO: 53, and SEQ ID NO: 54, respectively, and CDR1-3 of light chain variable region consisting of the amino acid sequences of SEQ ID NO: 56, SEQ ID NO: 57, and SEQ ID NO: 58, respectively (e.g., an antibody comprising the amino acid sequence of a heavy chain variable region represented by SEQ ID NO: 55 comprising the CDR1-3 of heavy chain variable region, and the amino acid sequence of a light chain variable region represented by SEQ ID NO: 59 comprising the CDR1-3 of light chain variable region).

The monoclonal antibody against CAPRIN-1 disclosed in WO2013/125654, wherein the antibody comprises CDR1-3 of heavy chain variable region consisting of the amino acid sequences of SEQ ID NO: 60, SEQ ID NO: 61, and SEQ ID NO: 62, respectively, and CDR1-3 of light chain variable region consisting of the amino acid sequences of SEQ ID NO: 64, SEQ ID NO: 65, and SEQ ID NO: 66, respectively (e.g., an antibody comprising the amino acid sequence of a heavy chain variable region represented by SEQ ID NO: 63 comprising the CDR1-3 of heavy chain variable region, and the amino acid sequence of a light chain variable region represented by SEQ ID NO: 67 comprising the CDR1-3 of light chain variable region).

The monoclonal antibody against CAPRIN-1 disclosed in WO2011/096517, wherein the antibody comprises the amino acid sequence of a heavy chain variable region represented by the amino acid sequence of SEQ ID NO: 68 and the amino acid sequence of a light chain variable region represented by the amino acid sequence of SEQ ID NO: 69.

The monoclonal antibody against CAPRIN-1 disclosed in WO2011/096528, wherein the antibody comprises the amino acid sequence of a heavy chain variable region represented by the amino acid sequence of SEQ ID NO: 70 and the amino acid sequence of a light chain variable region represented by the amino acid sequence of SEQ ID NO: 71; the antibody comprises the amino acid sequence of a heavy chain variable region represented by the amino acid sequence of SEQ ID NO: 72 and the amino acid sequence of a light chain variable region represented by the amino acid sequence of SEQ ID NO: 73; the antibody comprises the amino acid sequence of a heavy chain variable region represented by the amino acid sequence of SEQ ID NO: 74 and the amino acid sequence of a light chain variable region represented by the amino acid sequence of SEQ ID NO: 75; the antibody comprises the amino acid sequence of a heavy chain variable region represented by the amino acid sequence of SEQ ID NO: 76 and the amino acid sequence of a light chain variable region represented by the amino acid sequence of SEQ ID NO: 77; or the antibody comprises the amino acid sequence of a heavy chain variable region represented by the amino acid sequence of SEQ ID NO: 78 and the amino acid sequence of a light chain variable region represented by the amino acid sequence of SEQ ID NO: 79.

The monoclonal antibody against CAPRIN-1 disclosed in WO2011/096533, wherein the antibody comprises the amino acid sequence of a heavy chain variable region represented by the amino acid sequence of SEQ ID NO: 80 and the amino acid sequence of a light chain variable region represented by the amino acid sequence of SEQ ID NO: 81, or the antibody comprises the amino acid sequence of a heavy chain variable region represented by the amino acid sequence of SEQ ID NO: 82 and the amino acid sequence of a light chain variable region represented by the amino acid sequence of SEQ ID NO: 83.

The monoclonal antibody against CAPRIN-1 disclosed in WO2011/096534, wherein the antibody comprises the amino acid sequence of a heavy chain variable region represented by the amino acid sequence of SEQ ID NO: 84 and the amino acid sequence of a light chain variable region represented by the amino acid sequence of SEQ ID NO: 85, or the antibody comprises the amino acid sequence of a heavy chain variable region represented by the amino acid sequence of SEQ ID NO: 86 and the amino acid sequence of a light chain variable region represented by the amino acid sequence of SEQ ID NO: 87.

The monoclonal antibody against CAPRIN-1 disclosed in WO2010/016526, wherein the antibody comprises the amino acid sequence of a heavy chain variable region represented by the amino acid sequence of SEQ ID NO: 88 and the amino acid sequence of a light chain variable region represented by the amino acid sequence of SEQ ID NO: 89; the antibody comprises the amino acid sequence of a heavy chain variable region represented by the amino acid sequence of SEQ ID NO: 90 and the amino acid sequence of a light chain variable region represented by the amino acid sequence of SEQ ID NO: 91; the antibody comprises the amino acid sequence of a heavy chain variable region represented by the amino acid sequence of SEQ ID NO: 92 and the amino acid sequence of a light chain variable region represented by the amino acid sequence of SEQ ID NO: 93; the antibody comprises the amino acid sequence of a heavy chain variable region represented by the amino acid sequence of SEQ ID NO: 94 and the amino acid sequence of a light chain variable region represented by the amino acid sequence of SEQ ID NO: 95; the antibody comprises the amino acid sequence of a heavy chain variable region represented by the amino acid sequence of SEQ ID NO: 96 and the amino acid sequence of a light chain variable region represented by the amino acid sequence of SEQ ID NO: 97; the antibody comprises the amino acid sequence of a heavy chain variable region represented by the amino acid sequence of SEQ ID NO: 98 and the amino acid sequence of a light chain variable region represented by the amino acid sequence of SEQ ID NO: 99; or the antibody comprises the amino acid sequence of a heavy chain variable region represented by the amino acid sequence of SEQ ID NO: 100 and the amino acid sequence of a light chain variable region represented by the amino acid sequence of SEQ ID NO: 101.

The monoclonal antibody against CAPRIN-1 disclosed in WO2013/018894, wherein the antibody comprises the amino acid sequence of a heavy chain variable region represented by the amino acid sequence of SEQ ID NO: 102 and the amino acid sequence of a light chain variable region represented by the amino acid sequence of SEQ ID NO: 103, or the antibody comprises the amino acid sequence of a heavy chain variable region represented by the amino acid sequence of SEQ ID NO: 104 and the amino acid sequence of a light chain variable region represented by the amino acid sequence of SEQ ID NO: 105.

The monoclonal antibody against CAPRIN-1 disclosed in WO2013/018892, wherein the antibody comprises the amino acid sequence of a heavy chain variable region represented by the amino acid sequence of SEQ ID NO: 106 and the amino acid sequence of a light chain variable region represented by the amino acid sequence of SEQ ID NO: 107.

The monoclonal antibody against CAPRIN-1 disclosed in WO2013/018891, wherein the antibody comprises the amino acid sequence of a heavy chain variable region represented by the amino acid sequence of SEQ ID NO: 108 and the amino acid sequence of a light chain variable region represented by the amino acid sequence of SEQ ID NO: 109.

The monoclonal antibody against CAPRIN-1 disclosed in WO2013/018889, wherein the antibody comprises the amino acid sequence of a heavy chain variable region represented by the amino acid sequence of SEQ ID NO: 110 and the amino acid sequence of a light chain variable region represented by the amino acid sequence of SEQ ID NO: 111.

The monoclonal antibody against CAPRIN-1 disclosed in WO2013/018883, wherein the antibody comprises the amino acid sequence of a heavy chain variable region represented by the amino acid sequence of SEQ ID NO: 112 and the amino acid sequence of a light chain variable region represented by the amino acid sequence of SEQ ID NO: 113.

The monoclonal antibody against CAPRIN-1 disclosed in WO2013/125636, wherein the antibody comprises the amino acid sequence of a heavy chain variable region represented by the amino acid sequence of SEQ ID NO: 114 and the amino acid sequence of a light chain variable region represented by the amino acid sequence of SEQ ID NO: 115.

The monoclonal antibody against CAPRIN-1 disclosed in WO2013/125654, wherein the antibody comprises the amino acid sequence of a heavy chain variable region represented by the amino acid sequence of SEQ ID NO: 116 and the amino acid sequence of a light chain variable region represented by the amino acid sequence of SEQ ID NO: 117, or the antibody comprises the amino acid sequence of a heavy chain variable region represented by the amino acid sequence of SEQ ID NO: 118 and the amino acid sequence of a light chain variable region represented by the amino acid sequence of SEQ ID NO: 119.

The monoclonal antibody against CAPRIN-1 disclosed in WO2013/125630, wherein the antibody comprises the amino acid sequence of a heavy chain variable region represented by the amino acid sequence of SEQ ID NO: 120 and the amino acid sequence of a light chain variable region represented by the amino acid sequence of SEQ ID NO: 121.

The monoclonal antibody against CAPRIN-1 disclosed in WO2015/020212, wherein the antibody comprises the amino acid sequence of a heavy chain variable region represented by the amino acid sequence of SEQ ID NO: 122 and the amino acid sequence of a light chain variable region represented by the amino acid sequence of SEQ ID NO: 123; the antibody comprises the amino acid sequence of a heavy chain variable region represented by the amino acid sequence of SEQ ID NO: 124 and the amino acid sequence of a light chain variable region represented by the amino acid sequence of SEQ ID NO: 125; the antibody comprises the amino acid sequence of a heavy chain variable region represented by the amino acid sequence of SEQ ID NO: 126 and the amino acid sequence of a light chain variable region represented by the amino acid sequence of SEQ ID NO: 127; the antibody comprises the amino acid sequence of a heavy chain variable region represented by the amino acid sequence of SEQ ID NO: 128 and the amino acid sequence of a light chain variable region represented by the amino acid sequence of SEQ ID NO: 129; the antibody comprises the amino acid sequence of a heavy chain variable region represented by the amino acid sequence of SEQ ID NO: 130 and the amino acid sequence of a light chain variable region represented by the amino acid sequence of SEQ ID NO: 131; or the antibody comprises the amino acid sequence of a heavy chain variable region represented by the amino acid sequence of SEQ ID NO: 132 and the amino acid sequence of a light chain variable region represented by the amino acid sequence of SEQ ID NO: 133.

A base sequence was designed to express a heavy chain variable region comprising CDR1-3 of heavy chain variable region consisting of the amino acid sequences of SEQ ID NO: 36, SEQ ID NO: 37, and SEQ ID NO: 38, respectively, which are from one of the above-described anti-CAPRIN-1 monoclonal antibodies, and framework region comprising sequences of a human antibody; and the base sequence was inserted into a mammalian expression vector with an insert encoding a heavy chain constant region of human IgG1. Similarly, a base sequence was designed to express a light chain variable region comprising CDR1-3 of light chain variable region consisting of the amino acid sequences of SEQ ID NO: 40, SEQ ID NO: 41, and SEQ ID NO: 42, respectively, and framework region comprising sequences of a human antibody; and the base sequence was inserted into a mammalian expression vector with an insert encoding a light chain constant region of human IgG1. These two recombinant expression vectors were transferred to mammalian cells according to a conventional method, and a culture supernatant containing humanized monoclonal antibody #1 against CAPRIN-1 (humanized antibody #1) comprising CDR1-3 of heavy chain variable region consisting of the amino acid sequences of SEQ ID NO: 36, SEQ ID NO: 37, and SEQ ID NO: 38, respectively, and CDR1-3 of light chain variable region consisting of the amino acid sequences of SEQ ID NO: 40, SEQ ID NO: 41, and SEQ ID NO: 42, respectively, was obtained from the cells.

Similarly, a base sequence was designed to express a heavy chain variable region represented by SEQ ID NO: 47 comprising CDR1-3 of heavy chain variable region consisting of the amino acid sequences of SEQ ID NO: 44, SEQ ID NO: 45, and SEQ ID NO: 46, respectively, and framework region comprising sequences of a human antibody; and the base sequence was inserted into a mammalian expression vector with an inserted heavy chain constant region of human IgG1. Similarly, a base sequence was designed to express a heavy chain variable region represented by SEQ ID NO: 51 comprising CDR1-3 of light chain variable region consisting of the amino acid sequences of SEQ ID NO: 48, SEQ ID NO: 49, and SEQ ID NO: 50, respectively, and framework region comprising sequences of a human antibody; and the base sequence was inserted into a mammalian expression vector with an inserted heavy chain constant region of human IgG1. These two recombinant expression vectors were transferred to mammalian cells according to a conventional method, and a culture supernatant containing humanized anti-CAPRIN-1 monoclonal antibody #2 (humanized antibody #2) comprising CDR1-3 of heavy chain variable region consisting of the amino acid sequences of SEQ ID NO: 44, SEQ ID NO: 45, and SEQ ID NO: 46, respectively, and CDR1-3 of light chain variable region consisting of the amino acid sequences of SEQ ID NO: 48, SEQ ID NO: 49, and SEQ ID NO: 50, respectively, was obtained from the cells.

A culture supernatant containing humanized anti-CAPRIN-1 monoclonal antibody #3 (humanized antibody #3) comprising CDR1-3 of heavy chain variable region consisting of the amino acid sequences of SEQ ID NO: 52, SEQ ID NO: 53, and SEQ ID NO: 54, respectively, and CDR1-3 of light chain variable region consisting of the amino acid sequences of SEQ ID NO: 56, SEQ ID NO: 57, and SEQ ID NO: 58, respectively, was prepared in the same manner.

A culture supernatant containing humanized anti-CAPRIN-1 monoclonal antibody #4 (humanized antibody #4) comprising CDR1-3 of heavy chain variable region consisting of the amino acid sequences of SEQ ID NO: 60, SEQ ID NO: 61, and SEQ ID NO: 62, respectively, and CDR1-3 of light chain variable region consisting of the amino acid sequences of SEQ ID NO: 64, SEQ ID NO: 65, and SEQ ID NO: 66, respectively, was prepared in the same manner.

Culture supernatants containing the following humanized anti-CAPRIN-1 monoclonal antibodies #9 to #41 (humanized antibodies #9 to #41) were prepared in the same manner.

Humanized monoclonal antibody #9 (humanized antibody #9) comprising the amino acid sequence of a heavy chain variable region represented by the amino acid sequence of SEQ ID NO: 68 and the amino acid sequence of a light chain variable region represented by the amino acid sequence of SEQ ID NO: 69.

Humanized monoclonal antibody #10 (humanized antibody #10) comprising the amino acid sequence of a heavy chain variable region represented by the amino acid sequence of SEQ ID NO: 70 and the amino acid sequence of a light chain variable region represented by the amino acid sequence of SEQ ID NO: 71.

Humanized monoclonal antibody #11 (humanized antibody #11) comprising the amino acid sequence of a heavy chain variable region represented by the amino acid sequence of SEQ ID NO: 72 and the amino acid sequence of a light chain variable region represented by the amino acid sequence of SEQ ID NO: 73.

Humanized monoclonal antibody #12 (humanized antibody #12) comprising the amino acid sequence of a heavy chain variable region represented by the amino acid sequence of SEQ ID NO: 74 and the amino acid sequence of a light chain variable region represented by the amino acid sequence of SEQ ID NO: 75.

Humanized monoclonal antibody #13 (humanized antibody #13) comprising the amino acid sequence of a heavy chain variable region represented by the amino acid sequence of SEQ ID NO: 76 and the amino acid sequence of a light chain variable region represented by the amino acid sequence of SEQ ID NO: 77.

Humanized monoclonal antibody #14 (humanized antibody #14) comprising the amino acid sequence of a heavy chain variable region represented by the amino acid sequence of SEQ ID NO: 78 and the amino acid sequence of a light chain variable region represented by the amino acid sequence of SEQ ID NO: 79.

Humanized monoclonal antibody #15 (humanized antibody #15) comprising the amino acid sequence of a heavy chain variable region represented by the amino acid sequence of SEQ ID NO: 80 and the amino acid sequence of a light chain variable region represented by the amino acid sequence of SEQ ID NO: 81.

Humanized monoclonal antibody #16 (humanized antibody #16) comprising the amino acid sequence of a heavy chain variable region represented by the amino acid sequence of SEQ ID NO: 82 and the amino acid sequence of a light chain variable region represented by the amino acid sequence of SEQ ID NO: 83.

Humanized monoclonal antibody #17 (humanized antibody #17) comprising the amino acid sequence of a heavy chain variable region represented by the amino acid sequence of SEQ ID NO: 84 and the amino acid sequence of a light chain variable region represented by the amino acid sequence of SEQ ID NO: 85.

Humanized monoclonal antibody #18 (humanized antibody #18) comprising the amino acid sequence of a heavy chain variable region represented by the amino acid sequence of SEQ ID NO: 86 and the amino acid sequence of a light chain variable region represented by the amino acid sequence of SEQ ID NO: 87.

Humanized monoclonal antibody #19 (humanized antibody #19) comprising the amino acid sequence of a heavy chain variable region represented by the amino acid sequence of SEQ ID NO: 88 and the amino acid sequence of a light chain variable region represented by the amino acid sequence of SEQ ID NO: 89.

Humanized monoclonal antibody #20 (humanized antibody #20) comprising the amino acid sequence of a heavy chain variable region represented by the amino acid sequence of SEQ ID NO: 90 and the amino acid sequence of a light chain variable region represented by the amino acid sequence of SEQ ID NO: 91.

Humanized monoclonal antibody #21 (humanized antibody #21) comprising the amino acid sequence of a heavy chain variable region represented by the amino acid sequence of SEQ ID NO: 92 and the amino acid sequence of a light chain variable region represented by the amino acid sequence of SEQ ID NO: 93.

Humanized monoclonal antibody #22 (humanized antibody #22) comprising the amino acid sequence of a heavy chain variable region represented by the amino acid sequence of SEQ ID NO: 94 and the amino acid sequence of a light chain variable region represented by the amino acid sequence of SEQ ID NO: 95.

Humanized monoclonal antibody #23 (humanized antibody #23) comprising the amino acid sequence of a heavy chain variable region represented by the amino acid sequence of SEQ ID NO: 96 and the amino acid sequence of a light chain variable region represented by the amino acid sequence of SEQ ID NO: 97.

Humanized monoclonal antibody #24 (humanized antibody #24) comprising the amino acid sequence of a heavy chain variable region represented by the amino acid sequence of SEQ ID NO: 98 and the amino acid sequence of a light chain variable region represented by the amino acid sequence of SEQ ID NO: 99.

Humanized monoclonal antibody #25 (humanized antibody #25) comprising the amino acid sequence of a heavy chain variable region represented by the amino acid sequence of SEQ ID NO: 100 and the amino acid sequence of a light chain variable region represented by the amino acid sequence of SEQ ID NO: 101.

Humanized monoclonal antibody #26 (humanized antibody #26) comprising the amino acid sequence of a heavy chain variable region represented by the amino acid sequence of SEQ ID NO: 102 and the amino acid sequence of a light chain variable region represented by the amino acid sequence of SEQ ID NO: 103.

Humanized monoclonal antibody #27 (humanized antibody #27) comprising the amino acid sequence of a heavy chain variable region represented by the amino acid sequence of SEQ ID NO: 104 and the amino acid sequence of a light chain variable region represented by the amino acid sequence of SEQ ID NO: 105.

Humanized monoclonal antibody #28 (humanized antibody #28) comprising the amino acid sequence of a heavy chain variable region represented by the amino acid sequence of SEQ ID NO: 106 and the amino acid sequence of a light chain variable region represented by the amino acid sequence of SEQ ID NO: 107.

Humanized monoclonal antibody #29 (humanized antibody #29) comprising the amino acid sequence of a heavy chain variable region represented by the amino acid sequence of SEQ ID NO: 108 and the amino acid sequence of a light chain variable region represented by the amino acid sequence of SEQ ID NO: 109.

Humanized monoclonal antibody #30 (humanized antibody #30) comprising the amino acid sequence of a heavy chain variable region represented by the amino acid sequence of SEQ ID NO: 110 and the amino acid sequence of a light chain variable region represented by the amino acid sequence of SEQ ID NO: 111.

Humanized monoclonal antibody #31 (humanized antibody #31) comprising the amino acid sequence of a heavy chain variable region represented by the amino acid sequence of SEQ ID NO: 112 and the amino acid sequence of a light chain variable region represented by the amino acid sequence of SEQ ID NO: 113.

Humanized monoclonal antibody #32 (humanized antibody #32) comprising the amino acid sequence of a heavy chain variable region represented by the amino acid sequence of SEQ ID NO: 114 and the amino acid sequence of a light chain variable region represented by the amino acid sequence of SEQ ID NO: 115.

Humanized monoclonal antibody #33 (humanized antibody #33) comprising the amino acid sequence of a heavy chain variable region represented by the amino acid sequence of SEQ ID NO: 116 and the amino acid sequence of a light chain variable region represented by the amino acid sequence of SEQ ID NO: 117.

Humanized monoclonal antibody #34 (humanized antibody #34) comprising the amino acid sequence of a heavy chain variable region represented by the amino acid sequence of SEQ ID NO: 118 and the amino acid sequence of a light chain variable region represented by the amino acid sequence of SEQ ID NO: 119.

Humanized monoclonal antibody #35 (humanized antibody #35) comprising the amino acid sequence of a heavy chain variable region represented by the amino acid sequence of SEQ ID NO: 120 and the amino acid sequence of a light chain variable region represented by the amino acid sequence of SEQ ID NO: 121.

Humanized monoclonal antibody #36 (humanized antibody #36) comprising the amino acid sequence of a heavy chain variable region represented by the amino acid sequence of SEQ ID NO: 122 and the amino acid sequence of a light chain variable region represented by the amino acid sequence of SEQ ID NO: 123.

Humanized monoclonal antibody #37 (humanized antibody #37) comprising the amino acid sequence of a heavy chain variable region represented by the amino acid sequence of SEQ ID NO: 124 and the amino acid sequence of a light chain variable region represented by the amino acid sequence of SEQ ID NO: 125.

Humanized monoclonal antibody #38 (humanized antibody #38) comprising the amino acid sequence of a heavy chain variable region represented by the amino acid sequence of SEQ ID NO: 126 and the amino acid sequence of a light chain variable region represented by the amino acid sequence of SEQ ID NO: 127.

Humanized monoclonal antibody #39 (humanized antibody #39) comprising the amino acid sequence of a heavy chain variable region represented by the amino acid sequence of SEQ ID NO: 128 and the amino acid sequence of a light chain variable region represented by the amino acid sequence of SEQ ID NO: 129.

Humanized monoclonal antibody #40 (humanized antibody #40) comprising the amino acid sequence of a heavy chain variable region represented by the amino acid sequence of SEQ ID NO: 130 and the amino acid sequence of a light chain variable region represented by the amino acid sequence of SEQ ID NO: 131.

Humanized monoclonal antibody #41 (humanized antibody #41) comprising the amino acid sequence of a heavy chain variable region represented by the amino acid sequence of SEQ ID NO: 132 and the amino acid sequence of a light chain variable region represented by the amino acid sequence of SEQ ID NO: 133.

Additionally, among these anti-CAPRIN-1 monoclonal antibodies, a base sequence was designed based on humanized antibody #1 to express a heavy chain variable region comprising CDR1-3 consisting of the amino acid sequences of SEQ ID NO: 36, SEQ ID NO: 37, and SEQ ID NO: 38, respectively, and framework region comprising sequences of a human antibody. This base sequence was inserted into a mammalian expression vector with an inserted heavy chain constant region of human IgG1 in which serine (Ser) at amino acid position 239 by EU numbering is substituted with aspartic acid (Asp), and isoleucine (Ile) at amino acid position 332 by EU numbering is substituted with glutamic acid (Glu). Further, a base sequence was designed to express the amino acid sequence of a light chain variable region comprising CDR1-3 consisting of the amino acid sequences of SEQ ID NO: 40, SEQ ID NO: 41, and SEQ ID NO: 42, respectively, and framework region comprising sequences of a human antibody, and the base sequence was inserted into a mammalian expression vector with an inserted light chain constant region of human IgG1. These 2 recombinant expression vectors were transferred to mammalian cells according to a conventional method; and a culture supernatant containing humanized monoclonal antibody #5 (humanized antibody #5) against CAPRIN-1 composed of the full-length heavy chain amino acid sequence consisting of the heavy chain variable region designed above and the heavy chain constant region of human IgG1 in which serine (Ser) at amino acid position 239 by EU numbering is substituted with aspartic acid (Asp), and isoleucine (Ile) at amino acid position 332 by EU numbering is substituted with glutamic acid (Glu), and the full-length light chain amino acid sequence consisting of the light chain variable region designed above and the human light chain constant region, was obtained from the cells.

A culture supernatant containing humanized anti-CAPRIN-1 monoclonal antibody #6 (humanized antibody #6) composed of the amino acid sequence of the heavy chain variable region and the amino acid sequence of the light chain variable region of the humanized antibody #2 produced above was prepared in the same manner.

A culture supernatant containing humanized anti-CAPRIN-1 monoclonal antibody #7 (humanized antibody #7) composed of the amino acid sequence of the heavy chain variable region and the amino acid sequence of the light chain variable region of the humanized antibody #3 produced above was prepared in the same manner.

A culture supernatant containing humanized anti-CAPRIN-1 monoclonal antibody #8 (humanized antibody #8) composed of the amino acid sequence of the heavy chain variable region and the amino acid sequence of the light chain variable region of the humanized antibody #4 produced above was prepared in the same manner.

Culture supernatants containing each of humanized anti-CAPRIN-1 antibodies #42 to #74 (humanized antibodies #42 to #74) composed of the amino acid sequence of the heavy chain variable region and the amino acid sequence of the light chain variable region of each of the humanized antibodies #9 to #41 produced above were prepared in the same manner.

The obtained culture supernatants containing each of the humanized anti-CAPRIN-1 monoclonal antibodies #1 to #74 were subjected to purification using Hitrap Protein A Sepharose FF (GE Healthcare) according to a conventional method. The buffer was replaced with PBS(-). The resultant was filtered through a 0.22 µm filter (Merck Millipore Corp.) to prepare the humanized antibodies.

### (Example 3) Preparation of conjugate of anti-CAPRIN-1 antibody and dolastatin 10 derivative (MMAE) - 1

Conjugates of anti-CAPRIN-1 polyclonal antibodies #1 to #6 described in Example 1 with MMAE were prepared using maleimidocaproyl-valine-citrulline-p-aminobenzyloxycarbonyl (mc-val-Cit-PAB) as a linker. The preparation of these conjugates was carried out with reference to the method described in WO2014/012479.

After anti-CAPRIN-1 polyclonal antibody #1 described in Example 1 was prepared in PBS(-) at a concentration of 10 mg/ml, a solution of 0.45 mM TCEP-HCl dissolved in PBS(-) containing 2 mM EDTA was added at the molar ratio of the antibody to TCEP of 1:3, followed by reduction reaction at 25°C for 2 hours. The dolastatin derivative mc-vc-PAB-MMAE (HY-15575, MCE) dissolved in DMSO at 5 mM was added to the reduced solution at the molar ratio of the antibody to MMAE of 1 :7.5, followed by reaction at 25°C for 1 hour. After the reaction, the solution was centrifuged at 15,000 rpm for 5 minutes, and the supernatant was then collected. The unbound mc-vc-PAB-MMAE was removed using a gel filtration column (40k, ZEBA Spin desalting columns, Thermo Fischer Scientific, Inc.) and replaced with PBS(-). After removing of the unbound mc-vc-PAB-MMAE by the gel filtration column twice, the resultant was filtered through a 0.22 µm sterilization filter membrane to obtain a solution containing anti-CAPRIN-1 polyclonal antibody #1-mc-vc-PAB-MMAE of the present invention (Conjugate 1).

Similarly for anti-CAPRIN-1 polyclonal antibodies #2 to #6, solutions containing conjugates of mc-vc-PAB-MMAE (conjugate using anti-CAPRIN-1 polyclonal antibody #2: Conjugate 2; conjugate using anti-CAPRIN-1 polyclonal antibody #3: Conjugate 3; conjugate using anti-CAPRIN-1 polyclonal antibody #4: Conjugate 4; conjugate using anti-CAPRIN-1 polyclonal antibody #5: Conjugate 5; and conjugate using anti-CAPRIN-1 polyclonal antibody #6: Conjugate 6) were prepared. As for the rabbit control antibody described in Example 1 unreactive against the CAPRIN-1 protein, a solution containing a conjugate of the rabbit control antibody and the MMAE linker (Control conjugate 1) was also prepared in the same manner as described above.

In addition, a solution containing a conjugate with MMAE (Conjugate 7) was prepared using the humanized antibody #1, which is an anti-CAPRIN-1 monoclonal antibody described in Example 2, in the same manner as above. A solution containing a conjugate with MMAE (Conjugate 8) was prepared using the humanized antibody #2, which is an anti-CAPRIN-1 antibody described in Example 2, in the same manner. As described above, each of the solutions containing the following: a conjugate with MMAE (Conjugate 9) using humanized antibody #3 described in Example 2; a conjugate with MMAE (Conjugate 10) using humanized antibody #4; a conjugate with MMAE (Conjugate 11) using humanized antibody #5; a conjugate with MMAE (Conjugate 12) using humanized antibody #6; a conjugate with MMAE (Conjugate 13) using humanized antibody #7; a conjugate with MMAE (Conjugate 14) using humanized antibody #8; and solutions containing conjugates with MMAE (Conjugates 15 to 80) using humanized antibodies #9 to #74, were prepared and filtered solutions were prepared by filtering through a 0.22 µm filter (Merck Millipore Corp.).

A dolastatin 15 derivative having toxicity to cancer cells by the activity of inhibiting the formation of the microtubules or mitosis by directly acting on microtubules was synthesized as a comparative control according to known information (Cancer Chemotherapy and Pharmacology. 2012, 70, 439-449), and a comparative conjugate of the dolastatin 15 derivative and the antibody against CAPRIN-1 prepared in Examples 1 and 2 was prepared according to the above method.

### (Example 4) Preparation of conjugate of anti-CAPRIN-1 antibody and dolastatin 10 derivative (MMAE) - 2

Conjugates of anti-CAPRIN-1 polyclonal antibodies #1 to #6 described in Example 1 with MMAE were prepared using OSu-Glu-vc-PAB-MMAE and OSu-vc-PAB-MMAE.

After anti-CAPRIN-1 polyclonal antibody #1 described in Example 1 was prepared in PBS(-) at a concentration of 5 mg/ml, OSu-Glu-vc-PAB-MMAE (SET0100, Levena Biopharma) or OSu-vc-PAB-MMAE (CS-0106796, ChemScene) was added at the molar ratio of the antibody to MMAE of 1:3 to 6, followed by reaction at 20°C for 16 hours. After the reaction, the unbound drug-linker was removed using a gel filtration column (NAP-10 column, Cytiva) and replaced with PB S(-). After removing the unbound drug-linker by the gel filtration column twice, the resultant was filtered through a 0.22 µm sterilization filter membrane to obtain a solution containing anti-CAPRIN-1 polyclonal antibody #1 bound to the MMAE of the present invention (Conjugate 81).

Similarly for anti-CAPRIN-1 polyclonal antibodies #2 to #6, solutions containing conjugates using OSu-Glu-vc-PAAP-MMAE or OSu-vc-PABA-MMAE (conjugate using anti-CAPRIN-1 polyclonal antibody #2: Conjugate 82; conjugate using anti-CAPRIN-1 polyclonal antibody #3: Conjugate 83; conjugate using anti-CAPRIN-1 polyclonal antibody #4: Conjugate 84; conjugate using anti-CAPRIN-1 polyclonal antibody #5: Conjugate 85; and conjugate using anti-CAPRIN-1 polyclonal antibody #6: Conjugate 86) were prepared. As for the rabbit control antibody described in Example 1 unreactive against the CAPRIN-1 protein, a solution containing a conjugate of the rabbit control antibody and the MMAE linker (Control conjugate 2) was also prepared in the same manner as above.

In addition, a solution containing a conjugate with MMAE (Conjugate 87) was prepared using the humanized antibody #1, which is the anti-CAPRIN-1 monoclonal antibodies described in Example 2, in the same manner as above. A solution containing a conjugate with MMAE (Conjugate 88) was obtained using the humanized antibody #2, which is one of the anti-CAPRIN-1 antibodies described in Example 2, in the same manner. In the same manner as above, each of the solutions containing the following: a conjugate with MMAE (Conjugate 89) using humanized antibody #3 described in Example 2; a conjugate with MMAE (Conjugate 90) using humanized antibody #4; a solution containing a conjugate with MMAE (Conjugate 91) using humanized antibody #5; a solution containing a conjugate with MMAE (Conjugate 92) using humanized antibody #6; a solution containing a conjugate with MMAE (Conjugate 93) using humanized antibody #7; a solution containing a conjugate with MMAE (Conjugate 94) using humanized antibody #8; and solutions containing conjugates with MMAE (Conjugates 95 to 160) using humanized antibodies #9 to #74, were prepared, and filtered solutions were prepared by filtering through a 0.22 µm filter (Merck Millipore Corp.).

### (Example 5) Specific reactivity of conjugate against CAPRIN-1 protein and CAPRIN-1-expressing cancer cell

Conjugates 1 to 80 prepared in Example 3 and Conjugates 81 to 160 prepared in Example 4 were assayed for their specific reactivity against a CAPRIN-1 protein and their reactivity against the cell membrane surface of human cancer cells and mouse cancer cells that express a CAPRIN-1 protein.

The specific reactivity against the CAPRIN-1 protein was determined by ELISA. 1 µg/mL CAPRIN-1 protein solution was added at 100 µL/well to a 96-well plate, and the plate was left at 4°C for 18 hours. Each well was washed with PBS-T three times. Then, a 0.5% bovine serum albumin (BSA) solution was added at 400 µL/well, and the plate was left at room temperature for 3 hours. The solution was removed, and the wells were washed with 400 µL/well of PBS-T three times. Then, each of the solutions containing Conjugates 1 to 6, Conjugates 81 to 86, Control conjugate 1 and 2 was added at 100 µL/well, and the plate was left at room temperature for 2 hours. Each well was washed with PBS-T three times. Then, an HRP-labeled anti-rabbit antibody diluted 5000-fold with PBS was added at 100 µL/well, and the plate was left at room temperature for 1 hour. Each well was washed with PBS-T three times. Then, a TMB substrate solution was added at 100 µL/well, and the plate was left for 15 to 30 minutes for chromogenic reaction. After the color developed, the reaction was terminated by adding 1 N sulfuric acid at 100 µL/well, and the absorbance values at 450 nm and 595 nm were measured using an absorption spectrometer. As a result, Conjugates 1 to 6 and Conjugates 81 to 86 exhibited a higher absorbance value than that of Control conjugates 1 and 2 as negative controls and were found to specifically react against the CAPRIN-1 protein.

Similarly for Conjugates 7 to 80 and Conjugates 87 to 160, specific reactivity against the CAPRIN-1 protein were detected using HRP-labeled human antibodies by ELISA. As a result, Conjugates 7 to 80 and Conjugates 87 to 160 exhibited a higher absorbance value than that of human IgG (Sigma-Aldrich Corp) conjugated in the same manner as Examples 3 and 4 (Control conjugates 3 and 4, respectively) as a negative control, and were found to specifically react against the CAPRIN-1 protein.

Next, the reactivity against the cell membrane surface of CAPRIN-1-expressing cancer cells was verified by flow cytometry. 2 × 10⁵ cells of human breast cancer cells BT-474 (from ATCC) or mouse breast cancer cells 4T1 (from ATCC) were centrifuged in 1.5 mL microcentrifuge tubes. 100 µL of the solutions containing each of Conjugates 1 to 6, Conjugates 81 to 86, Control conjugate 1 and 2 was then added to separate tubes. The tube was left at 4°C for 1 hour. After washing with PBS, Alexa 488-labeled anti-rabbit IgG (H + L) diluted 100-fold with PBS(-) containing 0.5% FBS (0.5% FBS-PBS(-)) was added thereto, and the tube was left at 4°C for 1 hour. After washing with 0.5% FBS-PBS(-), the cells were reacted with BD Horizon Fixable Viability Stain (FVS) Reagents (FVS450, Becton, Dickinson and Company) to stain dead cells, and the fluorescence intensity was then measured using FACSFortessa^{™} (Becton, Dickinson and Company). As a result, Conjugates 1 to 6 and Conjugates 81 to 86, which were the conjugates of the anti-CAPRIN-1 polyclonal antibodies and MMAE, were found to exhibit higher fluorescence intensity than that of Control conjugates 1 and 2 as negative controls, and were thus shown, to strongly react against the cell surface of the human cancer cell BT474 expressing CAPRIN-1 was confirmed.

Similarly, Conjugates 7 to 80 and Conjugates 87 to 160, which were the conjugates of the anti-CAPRIN-1 monoclonal antibodies and MMAE, were detected for their specific reactivity against human cancer cells BT474 and mouse cancer cells 4T1 as CAPRIN-1-expressing cancer cells using Alexa 488-labeled human IgG (H + L) antibodies. As a result, Conjugates 7 to 80 and Conjugates 87 to 160, which were the conjugates of the anti-CAPRIN-1 polyclonal antibodies and MMAE, were found to exhibit higher fluorescence intensity than that of Control conjugates 3 and 4 as negative controls, and were thus shown to strongly react against the cell surface of the human cancer cells BT474 expressing CAPRIN-1.

Similarly, the reactivity of Conjugates 1 to 160 against the following various human cancer cells and mouse cancer cells was verified. The antitumor activity was evaluated against breast cancer cells (BT-474), colon cancer cells (HT-29), lung cancer cells (and A549), gastric cancer cells (NCI-N87), uterine cancer cells (HEC-1-A), prostate cancer cells (22Rv1), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), bile duct cancer cells (KKU213), fibrosarcoma cells (HT-1080), esophageal cancer cells (OE33), leukemia cells (OCI-AML5), lymphoma cells (Ramos), gallbladder cancer cells (TGBC14TKB), and melanoma cells (Malme-3M), which are human cancer cells found to express CAPRIN-1 on the cell membrane surface of the cancer cells, the human cancer cells found to express the CAPRIN-1 gene; and mouse kidney cancer cells (Renca) and mouse breast cancer cells (4T1), which are mouse cancer cells found to express the CAPRIN-1 gene. As a result of the verification, Conjugates 1 to 160, which were the conjugates of the anti-CAPRIN-1 antibodies and MMAE, exhibited stronger fluorescence intensities than that of Control conjugate 1 to 4 as negative controls for any of the cancer cells and were thus shown to strongly react against the cell membrane surface of the above cancer cells expressing CAPRIN-1.

### (Example 6) Antitumor activity of conjugate

Conjugates 1 to 6 and Conjugates 81 to 86 prepared using anti-CAPRIN-1 polyclonal antibodies #1 to #6 and Conjugates 7 to 80 and Conjugates 87 to 160 prepared using anti-CAPRIN-1 monoclonal antibodies in Examples 3 and 4 were evaluated for their *in vitro* antitumor activity against cancer cells. The antitumor activity was evaluated against breast cancer cells (BT-474), colon cancer cells (HT-29), lung cancer cells (and A549), gastric cancer cells (NCI-N87), uterine cancer cells (HEC-1-A), prostate cancer cells (22Rv1), pancreatic cancer cells (Panc10.5), liver cancer cells (Hep3B), ovarian cancer cells (SKOV3), kidney cancer cells (Caki-2), brain tumor cells (U-87MG), urinary bladder cancer cells (T24), bile duct cancer cells (KKU213), fibrosarcoma cells (HT-1080), esophageal cancer cells (OE33), leukemia cells (OCI-AML5), lymphoma cells (Ramos), gallbladder cancer cells (TGBC14TKB), and melanoma cells (Malme-3M), which are human cancer cells found to express CAPRIN-1 on the cell membrane surface of the cancer cells in Example 5; and mouse kidney cancer cells (Renca) and mouse breast cancer cells (4T1), which are mouse cancer cells found to express the CAPRIN-1 gene.

The cancer cells cultured by standard methods were seeded in a cell-culturable black 96-well plate (96 well optical Btm Pit PolymerBase Black w/Lid Cell Culture Sterile PS 165305, Thermo Fischer Scientific, Inc.) at 90 µL/well with 1 to 4 × 10⁴ cells per well and cultured at 5% CO₂ and 37°C for 18 to 24 hours in an incubator. Thereafter, Conjugates 1 to 14 and Conjugates 45 to 110 prepared in Example 3 and Conjugates 15 to 28 and Conjugates 111 to 176 prepared in Example 4 were added at 10 µL/well to have a concentration of 0.001 µg/mL to 100 µg/mL and incubated at 5% CO₂ and 37°C in an incubator. Cell survival rates were measured 3 days or 4 days after the start of the incubation using the Cell Titer Glow Luminescent Cell Viability Assay (#7573, (Promega K.K.). Substrate was added at 100 µL/well, and the plate was shaken for 2 minutes using a plate shaker and left to stand for 10 minutes. Then the luminescence signal intensity was measured using SpectraMax^{(R)} iD3 (Molecular Devices, LLC.) to calculate the ATP content of surviving cells.

As a result, Conjugates 1 to 6 and Conjugates 81 to 86, which were the conjugates of the antibodies against CAPRIN-1 and MMAE described in the present invention, exhibited stronger antitumor activity than that of Control conjugates 1 and 2 prepared using control antibodies unreactive against the CAPRIN-1 protein. Further, Conjugates 7 and 11, which were the conjugates of the antibodies against CAPRIN-1 and MMAE according to the present invention, had 60% or less of the surviving cancer cells against any of the cancer cells, relative to the negative control (non-conjugate group; 100%). The same results were also obtained for Conjugates 8 to 80, Conjugates 12 to 80, and Conjugates 87 to 160 as for Conjugates 7 and 11.

On the other hand, in the case of using the conjugate of the dolastatin 15 derivative prepared in Example 3, which was evaluated as the comparative group, the surviving cancer cells was 80% or more.

From the above results, it was revealed that the conjugate of the antibody against CAPRIN-1 and MMAE exhibited antitumor activity against cancer cells.

### (Example 7) Antitumor effect of conjugate on cancer-bearing mouse

The conjugates of the antibodies against CAPRIN-1 and the MMAE prepared in Examples 3 and 4 (Conjugates 7 to 80 and Conjugates 87 to 160) were evaluated for their *in vivo* antitumor effects on cancer-bearing mice. The conjugates of the present invention were examined for their antitumor effect using NOD-SCID mice in which human-derived cancer cells expressing the CAPRIN-1 protein were transplanted. 10⁷ cells per mouse of human colon cancer cells HT29 were mixed with Matrigel (Sigma-Aldrich Corp.) and subcutaneously transplanted to the mice, and were then grown until the tumor became 50 mm³ or larger to prepare cancer-bearing mice. HT29 expresses the CAPRIN-1 protein on the cell membrane surface, and as shown in Example 5, Conjugates 1 to 6 and Conjugates 81 to 86 prepared using anti-CAPRIN-1 polyclonal antibodies #1 to #6, and Conjugates 7 to 80 and Conjugates 87 to 160 prepared using anti-CAPRIN-1 monoclonal antibodies specifically bind to their cell membrane surface. Each of the conjugates were administered at 10 mg/kg to the tail veins of 10 cancer-bearing mice.

According to the method described in Example 3, a solution containing a conjugate of trastuzumab (Chugai Pharmaceutical Co., Ltd.) and MMAE was prepared so that the same amount of drugs was bound as the conjugate using the antibody against CAPRIN-1, and administered as a comparative control in the same amount as above to the cancer-bearing mice. HT29 expresses HER2 protein, which is a target antigen of trastuzumab, on the cell membrane surface. The conjugate of trastuzumab and MMAE specifically binds to HT29. The administration to the cancer-bearing mice was carried out twice a week.

As a negative control, PBS(-) was administered to the cancer-bearing mice.

The tumor sizes of the cancer-bearing mice after the administration were measured using calipers over time, and tumor volumes were calculated according to an ordinary method based on the formula: (Length of the major axis of tumor) × (Length of the minor axis of tumor)² × 0.5. As a result of the evaluation, in all the mice receiving Conjugates 7 to 14 prepared in Example 3 and Conjugates 87 to 94 prepared in Example 4 had tumor volumes at 7 days after the start of the administration were less than 30% when the tumor volume of the negative control was set at 100%. Thereafter, the tumor completely regressed 25 days after the start of the administration. As a result of similarly evaluating the *in vivo* antitumor effects of Conjugates 15 to 80 and Conjugates 95 to 160 on cancer-bearing mice, tumor volumes were less than 30% in all the mice. Further, in the cancer-bearing mice receiving the above conjugates, tumor volumes were less than 32% when the tumor volume of the cancer-bearing mice receiving the antibodies against CAPRIN-1 used in the conjugates alone was set at 100%.

On the other hand, the tumor volume of the mice receiving the solution containing the conjugate of trastuzumab and MMAE as a comparative control was 65% or more relative to the negative control. Further, in the cancer-bearing mice receiving the above conjugates, tumor volumes were less than 81% when the tumor volume of the cancer-bearing mice receiving the trastuzumab alone was set at 100%.

These evaluation results demonstrated that Conjugates 7 to 80 and Conjugates 87 to 160 prepared in Examples 3 and 4 using the antibodies against CAPRIN-1 exert a remarkably stronger antitumor effect than that of the negative control (untreated control group). These results also demonstrated that Conjugates 7 to 80 and Conjugates 87 to 160 have a remarkably stronger antitumor effect than that of the conjugate of trastuzumab and MMAE prepared as a comparative control.

## Claims

1. A conjugate of an antibody or a fragment thereof linked to dolastatin 10 or a derivative thereof, wherein the antibody or the fragment thereof has immunological reactivity against a CAPRIN-1 protein having an amino acid sequence represented by any of even-numbered SEQ ID NOs among SEQ ID NOs: 2 to 30 or an amino acid sequence having 80% or more sequence identity to said amino acid sequence.

2. The conjugate according to claim 1, wherein the antibody or the fragment thereof has immunological reactivity against a partial polypeptide of the CAPRIN-1 protein, wherein the partial polypeptide has an amino acid sequence represented by any of SEQ ID NOs: 31 to 35 and 296 to 299, 308, and 309 or an amino acid sequence having 80% or more sequence identity to said amino acid sequence.

3. The conjugate according to claim 1 or 2, wherein the antibody is a monoclonal antibody or a polyclonal antibody.

4. The conjugate according to any one of claims 1 to 3, wherein the antibody or the fragment thereof is any of the following (A) to (M):
(A) an antibody or a fragment thereof comprising a heavy chain variable region comprising complementarity-determining regions of SEQ ID NOs: 36, 37, and 38 (CDR1, CDR2, and CDR3, respectively), and a light chain variable region comprising complementarity-determining regions of SEQ ID NOs: 40, 41, and 42 (CDR1, CDR2, and CD3, respectively), and having immunological reactivity against the CAPRIN-1 protein,
(B) an antibody or a fragment thereof comprising a heavy chain variable region comprising complementarity-determining regions of SEQ ID NOs: 44, 45, and 46 (CDR1, CDR2, and CDR3, respectively), and a light chain variable region comprising complementarity-determining regions of SEQ ID NOs: 48, 49, and 50 (CDR1, CDR2, and CDR3, respectively), and having immunological reactivity against the CAPRIN-1 protein,
(C) an antibody or a fragment thereof comprising a heavy chain variable region comprising complementarity-determining regions of SEQ ID NOs: 52, 53, and 54 (CDR1, CDR2, and CDR3, respectively), and a light chain variable region comprising complementarity-determining regions of SEQ ID NOs: 56, 57, and 58 (CDR1, CDR2, and CDR3, respectively), and having immunological reactivity against the CAPRIN-1 protein,
(D) an antibody or a fragment thereof comprising a heavy chain variable region comprising complementarity-determining regions of SEQ ID NOs: 60, 61, and 62 (CDR1, CDR2, and CDR3, respectively), and a light chain variable region comprising complementarity-determining regions of SEQ ID NOs: 64, 65, and 66 (CDR1, CDR2, and CDR3, respectively), and having immunological reactivity against the CAPRIN-1 protein,
(E) an antibody or a fragment thereof comprising a heavy chain variable region comprising complementarity-determining regions of SEQ ID NOs: 170, 171, and 172 (CDR1, CDR2, and CDR3, respectively), and a light chain variable region comprising complementarity-determining regions of SEQ ID NOs: 173, 174, and 175 (CDR1, CDR2, and CDR3, respectively), and having immunological reactivity against the CAPRIN-1 protein,
(F) an antibody or a fragment thereof comprising a heavy chain variable region comprising complementarity-determining regions of SEQ ID NOs: 176, 177, and 178 (CDR1, CDR2, and CDR3, respectively), and a light chain variable region comprising complementarity-determining regions of SEQ ID NOs: 179, 180, and 181 (CDR1, CDR2, and CDR3, respectively), and having immunological reactivity against the CAPRIN-1 protein,
(G) an antibody or a fragment thereof comprising a heavy chain variable region comprising complementarity-determining regions of SEQ ID NOs: 182, 183, and 184 (CDR1, CDR2, and CDR3, respectively), and a light chain variable region comprising complementarity-determining regions of SEQ ID NOs: 185, 186, and 187 (CDR1, CDR2, and CDR3, respectively), and having immunological reactivity against the CAPRIN-1 protein,
(H) an antibody or a fragment thereof comprising a heavy chain variable region comprising complementarity-determining regions of SEQ ID NOs: 188, 189, and 190 (CDR1, CDR2, and CDR3, respectively), and a light chain variable region comprising complementarity-determining regions of SEQ ID NOs: 191, 192, and 193 (CDR1, CDR2, and CDR3, respectively), and having immunological reactivity against the CAPRIN-1 protein,
(I) an antibody or a fragment thereof comprising a heavy chain variable region comprising complementarity-determining regions of SEQ ID NOs: 146, 147, and 148 (CDR1, CDR2, and CDR3, respectively), and a light chain variable region comprising complementarity-determining regions of SEQ ID NOs: 149, 150, and 151 (CDR1, CDR2, and CDR3, respectively), and having immunological reactivity against the CAPRIN-1 protein,
(J) an antibody or a fragment thereof comprising a heavy chain variable region comprising complementarity-determining regions of SEQ ID NOs: 272, 273, and 274 (CDR1, CDR2, and CDR3, respectively), and a light chain variable region comprising complementarity-determining regions of SEQ ID NOs: 275, 276, and 277 (CDR1, CDR2, and CDR3, respectively), and having immunological reactivity against the CAPRIN-1 protein,
(K) an antibody or a fragment thereof comprising a heavy chain variable region comprising complementarity-determining regions of SEQ ID NOs: 290, 291, and 292 (CDR1, CDR2, and CDR3, respectively), and a light chain variable region comprising complementarity-determining regions of SEQ ID NOs: 293, 294, and 295 (CDR1, CDR2, and CDR3, respectively), and having immunological reactivity against the CAPRIN-1 protein,
(L) an antibody or a fragment thereof comprising a heavy chain variable region comprising complementarity-determining regions of SEQ ID NOs: 301, 302, and 303 (CDR1, CDR2, and CDR3, respectively), and a light chain variable region comprising complementarity-determining regions of SEQ ID NOs: 305, 306, and 307 (CDR1, CDR2, and CDR3, respectively), and having immunological reactivity against the CAPRIN-1 protein, and
(M) an antibody or a fragment thereof comprising a heavy chain variable region comprising complementarity-determining regions of SEQ ID NOs: 134, 135, and 136 (CDR1, CDR2, and CDR3, respectively), and a light chain variable region comprising complementarity-determining regions of SEQ ID NOs: 137, 138, and 139 (CDR1, CDR2, and CDR3, respectively), and having immunological reactivity against the CAPRIN-1 protein.

5. The conjugate according to any one of claims 1 to 4, wherein the antibody or the fragment thereof is any of the following (a) to (al):
(a) an antibody or a fragment thereof, comprising a heavy chain variable region comprising the amino acid sequence of SEQ ID NO: 39 and a light chain variable region comprising the amino acid sequence of SEQ ID NO: 43,
(b) an antibody or a fragment thereof, comprising a heavy chain variable region comprising the amino acid sequence of SEQ ID NO: 47 and a light chain variable region comprising the amino acid sequence of SEQ ID NO: 51,
(c) an antibody or a fragment thereof, comprising a heavy chain variable region comprising the amino acid sequence of SEQ ID NO: 55 and a light chain variable region comprising the amino acid sequence of SEQ ID NO: 59,
(d) an antibody or a fragment thereof, comprising a heavy chain variable region comprising the amino acid sequence of SEQ ID NO: 63 and a light chain variable region comprising the amino acid sequence of SEQ ID NO: 67,
(e) an antibody or a fragment thereof, comprising a heavy chain variable region comprising the amino acid sequence of SEQ ID NO: 68 and a light chain variable region comprising the amino acid sequence of SEQ ID NO: 69,
(f) an antibody or a fragment thereof, comprising a heavy chain variable region comprising the amino acid sequence of SEQ ID NO: 70 and a light chain variable region comprising the amino acid sequence of SEQ ID NO: 71,
(g) an antibody or a fragment thereof, comprising a heavy chain variable region comprising the amino acid sequence of SEQ ID NO: 72 and a light chain variable region comprising the amino acid sequence of SEQ ID NO: 73,
(h) an antibody or a fragment thereof, comprising a heavy chain variable region comprising the amino acid sequence of SEQ ID NO: 74 and a light chain variable region comprising the amino acid sequence of SEQ ID NO: 75,
(i) an antibody or a fragment thereof, comprising a heavy chain variable region comprising the amino acid sequence of SEQ ID NO: 76 and a light chain variable region comprising the amino acid sequence of SEQ ID NO: 77,
(j) an antibody or a fragment thereof, comprising a heavy chain variable region comprising the amino acid sequence of SEQ ID NO: 78 and a light chain variable region comprising the amino acid sequence of SEQ ID NO: 79,
(k) an antibody or a fragment thereof, comprising a heavy chain variable region comprising the amino acid sequence of SEQ ID NO: 80 and a light chain variable region comprising the amino acid sequence of SEQ ID NO: 81,
(l) an antibody or a fragment thereof, comprising a heavy chain variable region comprising the amino acid sequence of SEQ ID NO: 82 and a light chain variable region comprising the amino acid sequence of SEQ ID NO: 83,
(m) an antibody or a fragment thereof, comprising a heavy chain variable region comprising the amino acid sequence of SEQ ID NO: 84 and a light chain variable region comprising the amino acid sequence of SEQ ID NO: 85,
(n) an antibody or a fragment thereof, comprising a heavy chain variable region comprising the amino acid sequence of SEQ ID NO: 86 and a light chain variable region comprising the amino acid sequence of SEQ ID NO: 87,
(o) an antibody or a fragment thereof, comprising a heavy chain variable region comprising the amino acid sequence of SEQ ID NO: 88 and a light chain variable region comprising the amino acid sequence of SEQ ID NO: 89,
(p) an antibody or a fragment thereof, comprising a heavy chain variable region comprising the amino acid sequence of SEQ ID NO: 90 and a light chain variable region comprising the amino acid sequence of SEQ ID NO: 91,
(q) an antibody or a fragment thereof, comprising a heavy chain variable region comprising the amino acid sequence of SEQ ID NO: 92 and a light chain variable region comprising the amino acid sequence of SEQ ID NO: 93,
(r) an antibody or a fragment thereof, comprising a heavy chain variable region comprising the amino acid sequence of SEQ ID NO: 94 and a light chain variable region comprising the amino acid sequence of SEQ ID NO: 95,
(s) an antibody or a fragment thereof, comprising a heavy chain variable region comprising the amino acid sequence of SEQ ID NO: 96 and a light chain variable region comprising the amino acid sequence of SEQ ID NO: 97,
(t) an antibody or a fragment thereof, comprising a heavy chain variable region comprising the amino acid sequence of SEQ ID NO: 98 and a light chain variable region comprising the amino acid sequence of SEQ ID NO: 99,
(u) an antibody or a fragment thereof, comprising a heavy chain variable region comprising the amino acid sequence of SEQ ID NO: 100 and a light chain variable region comprising the amino acid sequence of SEQ ID NO: 101,
(v) an antibody or a fragment thereof, comprising a heavy chain variable region comprising the amino acid sequence of SEQ ID NO: 102 and a light chain variable region comprising the amino acid sequence of SEQ ID NO: 103,
(w) an antibody or a fragment thereof, comprising a heavy chain variable region comprising the amino acid sequence of SEQ ID NO: 104 and a light chain variable region comprising the amino acid sequence of SEQ ID NO: 105,
(x) an antibody or a fragment thereof, comprising a heavy chain variable region comprising the amino acid sequence of SEQ ID NO: 106 and a light chain variable region comprising the amino acid sequence of SEQ ID NO: 107,
(y) an antibody or a fragment thereof, comprising a heavy chain variable region comprising the amino acid sequence of SEQ ID NO: 108 and a light chain variable region comprising the amino acid sequence of SEQ ID NO: 109,
(z) an antibody or a fragment thereof, comprising a heavy chain variable region comprising the amino acid sequence of SEQ ID NO: 110 and a light chain variable region comprising the amino acid sequence of SEQ ID NO: 111,
(aa) an antibody or a fragment thereof, comprising a heavy chain variable region comprising the amino acid sequence of SEQ ID NO: 112 and a light chain variable region comprising the amino acid sequence of SEQ ID NO: 113,
(ab) an antibody or a fragment thereof, comprising a heavy chain variable region comprising the amino acid sequence of SEQ ID NO: 114 and a light chain variable region comprising the amino acid sequence of SEQ ID NO: 115,
(ac) an antibody or a fragment thereof, comprising a heavy chain variable region comprising the amino acid sequence of SEQ ID NO: 116 and a light chain variable region comprising the amino acid sequence of SEQ ID NO: 117,
(ad) an antibody or a fragment thereof, comprising a heavy chain variable region comprising the amino acid sequence of SEQ ID NO: 118 and a light chain variable region comprising the amino acid sequence of SEQ ID NO: 119,
(ae) an antibody or a fragment thereof, comprising a heavy chain variable region comprising the amino acid sequence of SEQ ID NO: 120 and a light chain variable region comprising the amino acid sequence of SEQ ID NO: 121,
(at) an antibody or a fragment thereof, comprising a heavy chain variable region comprising the amino acid sequence of SEQ ID NO: 122 and a light chain variable region comprising the amino acid sequence of SEQ ID NO: 123,
(ag) an antibody or a fragment thereof, comprising a heavy chain variable region comprising the amino acid sequence of SEQ ID NO: 124 and a light chain variable region comprising the amino acid sequence of SEQ ID NO: 125,
(ah) an antibody or a fragment thereof, comprising a heavy chain variable region comprising the amino acid sequence of SEQ ID NO: 126 and a light chain variable region comprising the amino acid sequence of SEQ ID NO: 127,
(ai) an antibody or a fragment thereof, comprising a heavy chain variable region comprising the amino acid sequence of SEQ ID NO: 128 and a light chain variable region comprising the amino acid sequence of SEQ ID NO: 129,
(aj) an antibody or a fragment thereof, comprising a heavy chain variable region comprising the amino acid sequence of SEQ ID NO: 130 and a light chain variable region comprising the amino acid sequence of SEQ ID NO: 131,
(ak) an antibody or a fragment thereof, comprising a heavy chain variable region comprising the amino acid sequence of SEQ ID NO: 132 and a light chain variable region comprising the amino acid sequence of SEQ ID NO: 133, and
(al) an antibody or a fragment thereof, comprising a heavy chain variable region comprising the amino acid sequence of SEQ ID NO: 300 and a light chain variable region comprising the amino acid sequence of SEQ ID NO: 304.

6. The conjugate according to any one of claims 1 to 5, wherein the antibody is a human antibody, a humanized antibody, a chimeric antibody, or a single-chain antibody.

7. The conjugate according to any one of claims 1 to 6, wherein the antibody or the fragment thereof is linked to the dolastatin 10 or the derivative thereof via a linker.

8. The conjugate according to any one of claims 1 to 7, wherein the dolastatin 10 derivative is monomethyl auristatin E (MMAE), monomethyl auristatin F (MMAF), or a derivative thereof.

9. A pharmaceutical composition for treatment and/or prevention of a cancer, comprising the conjugate according to any one of claims 1 to 8 as an active ingredient.

10. The pharmaceutical composition according to claim 9, further comprising one type or two or more types of antitumor agents together or separately in combination.

11. The pharmaceutical composition according to claim 9 or 10, wherein the cancer is a cancer expressing a CAPRIN-1 protein on the cell membrane surface.

12. The pharmaceutical composition according to any one of claims 9 to 11, wherein the cancer is breast cancer, kidney cancer, pancreatic cancer, colon cancer, lung cancer, brain tumor, gastric cancer, uterine cancer, ovarian cancer, prostate cancer, bladder cancer, esophageal cancer, leukemia, lymphoma, liver cancer, gallbladder cancer, bile duct cancer, sarcoma, mastocytoma, melanoma, adrenocortical carcinoma, Ewing's tumor, Hodgkin's lymphoma, mesothelioma, multiple myeloma, testicular cancer, thyroid cancer, head and neck cancer, urothelial carcinoma, renal cell carcinoma, colorectal cancer, gastroesophageal junction cancer, hepatocellular carcinoma, glioblastoma, primary central nervous system lymphoma, primary testicular lymphoma, biliary tract cancer, sarcoma, fibrosarcoma, basal cell carcinoma, Paget's disease, or skin cancer.

13. A method for treating and/or preventing a cancer, comprising administering the conjugate according to any one of claims 1 to 8 to a subject.

14. A method for treating and/or preventing a cancer, comprising administering the conjugate according to any one of claims 1 to 8 and one type or two or more types of antitumor agents together or separately in combination to a subject.
